# EUROPEAN PATENT SPECIFICATION

(11) **EP 2 967 887 B1**
(45) Date of publication and mention of the grant of the patent: **09.05.2018**
(21) Application number: 14768295.9
(22) Date of filing: 14.03.2014
(51) Int. Cl.: A61F 2/38, A61F 2/30, A61B 17/15, A61B 17/16

(54) **POSTERIOR-STABILIZED KNEE IMPLANT COMPONENTS AND INSTRUMENTS**
KOMPONENTEN UND INSTRUMENTE FÜR EIN VON HINTEN STABILISIERTES KNIEIMPLANTAT
COMPOSANTS POUR IMPLANT DU GENOU À STABILISATION POSTÉRIEURE ET INSTRUMENTS ASSOCIÉS

(30) Priority: 15.03.2013 US 201361801009 P
(43) Date of publication of application: 20.01.2016
(73) Proprietor: ConforMIS, Inc., Billerica, MA 01821 (US)
(72) Inventor: SLAMIN, John, Wrentham, MA 02093 (US); BOJARSKI, Raymond, Attleboro, MA 02703 (US); STEINES, Daniel, Lexington, MA 02421 (US); LANG, Philipp, Lexington, MA 02421 (US); WONG, Terrance, Needham, MA 02492 (US); FITZ, Wolfgang, Sherborn, MA 01770 (US)
(74) Representative: Grund, Martin
(86) International application number: PCT/US2014/027446
(87) International publication number: WO 2014/152533

(56) References cited:
- WO-A2-2011/075697
- US-A1- 2008 140 212
- US-A1- 2009 326 665
- US-A1- 2010 042 224
- US-A1- 2012 197 409
- US-A1- 2012 209 394
- US-A1- 2012 232 671
- US-B1- 6 325 828

## Description

### RELATED APPLICATIONS

### TECHNICAL FIELD

The present application relates to articular repair systems (e.g., resection cut strategy, guide tools, and implant components) as described in, for example, U.S. Patent Application Serial No. 13/397,457, entitled "Patient-Adapted and Improved Orthopedic Implants, Designs And Related Tools," filed February 15, 2012, and published as U.S. Patent Publication No. 2012-0209394. In particular, various embodiments disclosed herein provide improved features for knee joint articular repair systems designed for posterior stabilization, including patient-adapted (e.g., patient-specific and/or patient-engineered) features.

### BRIEF DESCRIPTION OF THE DRAWINGS

In the accompanying drawings, unless otherwise denoted herein, "M" and "L" in certain figures indicate medial and lateral sides of the view, respectively; "A" and "P" in certain figures indicate anterior and posterior sides of the view, respectively; and "S" and "I" in certain figures indicate superior and inferior sides of the view, respectively.
**FIGS. 1A** and **B** are perspective views of an exemplary posterior-stabilized femoral implant component;
**FIGS. 2A** and **2B** are side views of exemplary posterior-stabilized implant systems;
**FIGS. 3A** through **3K** depict various patient-adapted femoral intercondylar box embodiments;
**FIGS. 3L** through **3P** depict various cross-sections of the internal surfaces of various intercondylar box embodiments;
**FIGS. 4A-4R** illustrate sagittal cross-sections of femoral components with exemplary cams features;
**FIGS. 5A** and **5B** depict coronal cross-sections of exemplary post embodiments;
**FIGS. 6A** and **6B** depict perspective views of exemplary posterior-stabilized tibial components;
**FIG. 7A** is a perspective view of a posterior-stabilized femoral implant component with a cam tongue;
**FIGS. 7B** through **7F** are sagittal cross-section views of exemplary posterior-stabilized implant components with a cam tongue;
**FIG. 8** is a perspective view of a patient-adapted femoral jig;
**FIG. 9** is a perspective view of a patient-adapted femoral jig for a posterior-stabilized implant;
**FIG. 10** is a top view of a posterior-stabilized tibial implant component;
**FIG. 11** is a sagittal cross-section of posterior-stabilized femoral and tibial implant components;
**FIG. 12A** is a perspective view of an exemplary tibial post embodiment;
**FIG. 12B** depicts transverse cross-sections of the tibial post of **FIG. 12A****;**
**FIGS. 13A** and **13B** are sagittal cross-sections of exemplary posterior-stabilized tibial implant post embodiments;
**FIG. 14** is a top view of a two-piece posterior-stabilized tibial implant component embodiment;
**FIG. 15** is a top view of a two-piece tibial implant component embodiment; and
**FIGS. 16** through **27B** depict various sagittal cross-sectional views of modeled relationships of femoral and tibial implant components for designing a femoral cam.

### DETAILED DESCRIPTION

In this application, the use of the singular includes the plural unless specifically stated otherwise. Furthermore, the use of the term "including," as well as other forms, such as "includes" and "included," is not limiting. Also, terms such as "element" or "component" encompass both elements and components comprising one unit and elements and components that comprise more than one subunit, unless specifically stated otherwise. Also, the use of the term "portion" may include part of a moiety or the entire moiety.

The section headings used herein are for organizational purposes only and are not to be construed as limiting the subject matter described or the combination of features and/or embodiments described under one heading with features and/or embodiments described under another heading.

### Selecting and/or Designing a Patient-Adapted Implant Component

As described herein, an implant (also referred to as an "implant system") can include one or more implant components, which, can each include one or more patient-specific features, one or more patient-engineered features, and one or more standard (e.g., off-the-shelf, non-patient-specific) features. Moreover, an implant system can include one or more patient-adapted (e.g., patient-specific and/or patient-engineered) implant components and one or more standard implant components.

Using patient-specific information and measurements, and selected parameters and parameter thresholds, an implant component, resection cut strategy, and/or guide tool can be selected (e.g., from a library) and/or designed (e.g., virtually designed and manufactured) to have one or more patient-adapted features. In certain embodiments, one or more features of an implant component (and, optionally, one or more features of a resection cut strategy and/or guide tool) are selected for a particular patient based on patient-specific data and desired parameter targets or thresholds. For example, an implant component or implant component features can be selected from a virtual library of implant components and/or component features to include one or more patient-specific features and/or optimized features for a particular patient. Alternatively or in addition, an implant component can be selected from an actual library of implant components to include one or more patient-specific features and/or optimized features for the particular patient.

In some embodiments, the process of selecting an implant component can also include selecting one or more component features that optimizes fit with another implant component. In particular, for an implant that includes a first implant component and a second implant component that engage, for example, at a joint interface, selection of the second implant component can include selecting a component having a surface that provides best fit to the engaging surface of the first implant component. For example, for a knee implant that includes a femoral implant component and a tibial implant component, one or both of the components can be selected based, at least in part, on the fit of the outer (e.g., joint-facing) surface with the outer surface of the other component. The fit assessment can include, for example, selecting one or both of the medial and lateral tibial grooves (e.g., joint-facing articular bearing surfaces) on the tibial component and/or one or both of the medial and lateral condyles on the femoral component that substantially negatively-matches the fit or optimizes engagement in one or more dimensions, for example, in the coronal and/or sagittal dimensions. For example, a surface shape of a non-metallic component that best matches the dimensions and shape of an opposing metallic or ceramic or other hard material suitable for an implant component. By performing this component matching, component wear can be reduced.

For example, if a metal backed tibial component is used with a polyethylene insert or if an all polyethylene tibial component is used, the polyethylene can have one or two curved portions typically designed to mate with the femoral component in a low friction form. This mating can be optimized by selecting a polyethylene insert that is optimized or achieves an optimal fit with regard to one or more of: depth of the concavity, width of the concavity, length of the concavity, radius or radii of curvature of the concavity, and/or distance between two (e.g., medial and lateral) concavities. For example, the distance between a medial tibial concavity and a lateral tibial concavity can be selected so that it matches or approximates the distance between a medial and a lateral implant condyle component.

Not only the distance between two concavities, but also the radius / radii of curvature can be selected or designed so that it best matches the radius / radii of curvature on the femoral component. A medial and a lateral femoral condyle and opposite tibial component(s) can have a single radius of curvature in one or more dimensions, e.g., a coronal plane. They can also have multiple radii of curvature. The radius or radii of curvature on the medial condyle and/or medial tibial component can be different from that / those on a lateral condyle and/or lateral tibial component.

In various embodiments, implant bearing surfaces can be patient-adapted by combining patient-specific with standard features. For example the bearing surface of a femoral implant can have a patient-specific curvature in one direction and a standard curvature in another direction. One way to construct such a bearing surface is to generate one or more patient-specific curves substantially in a first direction (e.g., substantially in the sagittal plane). These curves can be derived directly from the patient's 2D or 3D images such as CT or MRI scans or radiographs. The curves may also be constructed using measurements derived from the patient's anatomy, such as curvature radii or dimensions. In some embodiments, these curves may be refined or optimized (e.g., smoothed). Once the patient-specific curves for the first direction have been constructed, a set of standard cross section profile curves can be calculated in the second direction along the patient-specific curves (e.g., multiple curves essentially transverse to the sagittal curves). Each of the cross section profile curves can be the same. The curves can also be rotated with respect to each other. The standard properties of the cross section profile curves such as the curvature radius can change in a step by step fashion from profile to profile. The profile curves can consist of standard segments, e.g., segments with a standard curvature radius. Different segments may have different curvature radii. The segments can be convex or concave. They can be connected to form smooth transitions between the segments. Once the cross section profile curves have been defined, the bearing surface (e.g., joint-facing surfaces) can be constructed, for example using a sweep operation, wherein the cross section profile curves are moved along the paths of the patient-specific curves to form a continuous surface.

Furthermore, in select high flexion designs, one or more of the posterior condyle curvature, implant thickness, edge thickness, bone cut orientation, and bone cut depth, can be adapted to maximize flexion. For example, the posterior bone cut can be offset more anteriorly for a given minimum thickness of the implant. This anterior offsetting of the posterior cut can be combined with a taper of the posterior implant bearing surface. Other strategies to enhance a patient's deep knee flexion include adding or extending the implant component posteriorly, at the end bearing surface in high flexion. By extending the bearing surface the knee can be flexed more deeply. Accordingly, in certain embodiments, the posterior edge and/or posterior bearing surface is patient-engineered to enhance deep knee flexion for the particular patient. These designs can be accompanied by corresponding designs on the tibial plateau, for example by change posterior insert height or slope or curvature relative to the corresponding femoral radius on the posterior condyle.

### Posterior Stabilized Articular Repair Systems

In addition to implant component features described above and in U.S. Patent Publication No. 2012-0209394, certain embodiments can include features associated with procedures that involve sacrificing one or more of the cruciate ligaments (e.g., the posterior cruciate ligament (PCL) and/or the anterior cruciate ligament (ACL)). For example, some embodiments may include features intended to function, at least in part, as a substitute for a patient's sacrificed PCL. Articular repair systems that include such features are commonly referred to as "posterior-stabilizing" (or "PS") systems. Accordingly, features intended, at least in part, individually or collectively, to substitute for, and/or compensate for the lack of, a patient's PCL and/or ACL are referred to herein generally as "posterior stabilizing" features or elements.

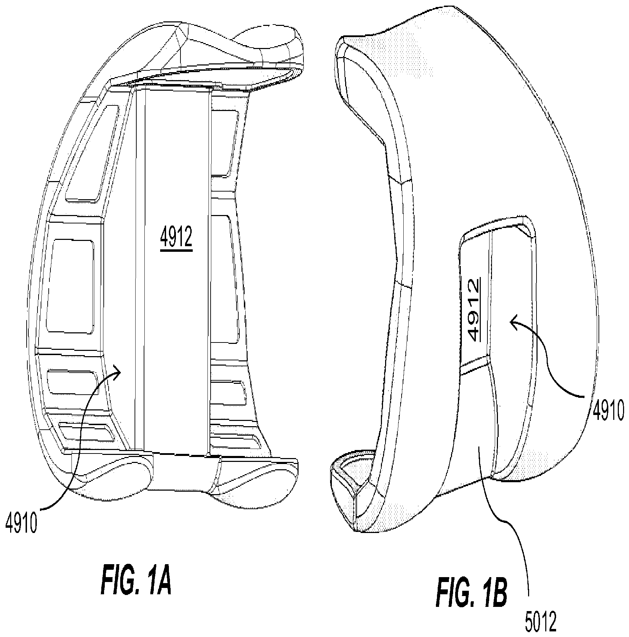

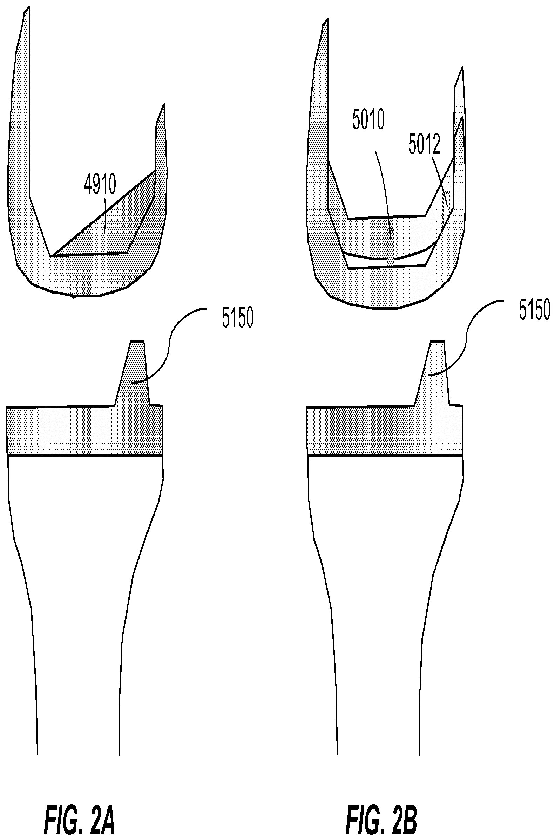

Posterior stabilizing features can include, for example, an intercondylar box (which may also be referred to herein as a "housing" or "receptacle") 4910, as shown in FIGS. 1A and 1B; an intercondylar cam (which may also be referred to herein as a "bar" or "keel") 5010, 5012, as shown in FIGS. 2A and 2B; and a tibial post (which may also be referred to herein as a "projection" or "spine") 5150. For example, as shown in FIGS. 2A and 2B, an intercondylar box and/or cam(s) of a femoral implant component may be configured to engage a post 5150 on a tibial implant component, which may thereby stabilize the joint through at least a portion of flexion or extension. Various embodiments of posterior stabilizing features and implant components are described in further detail below.

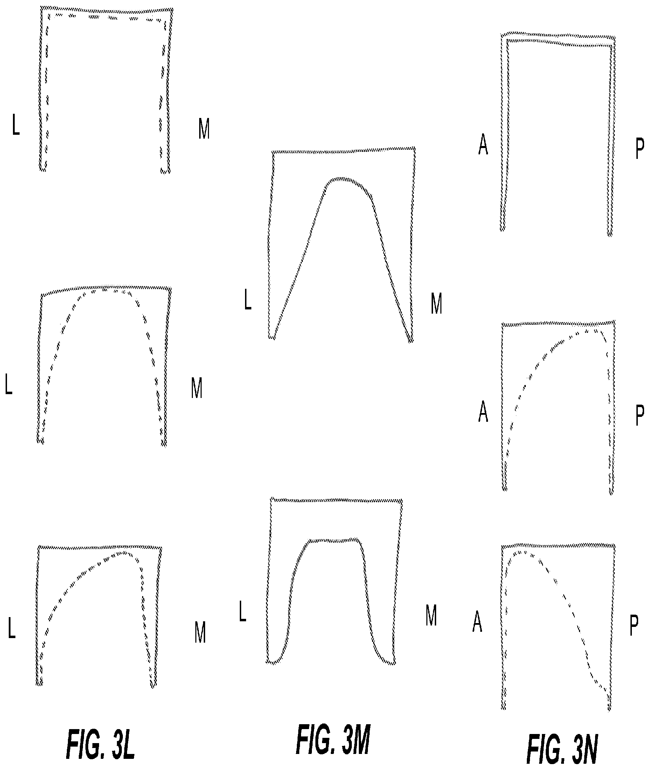

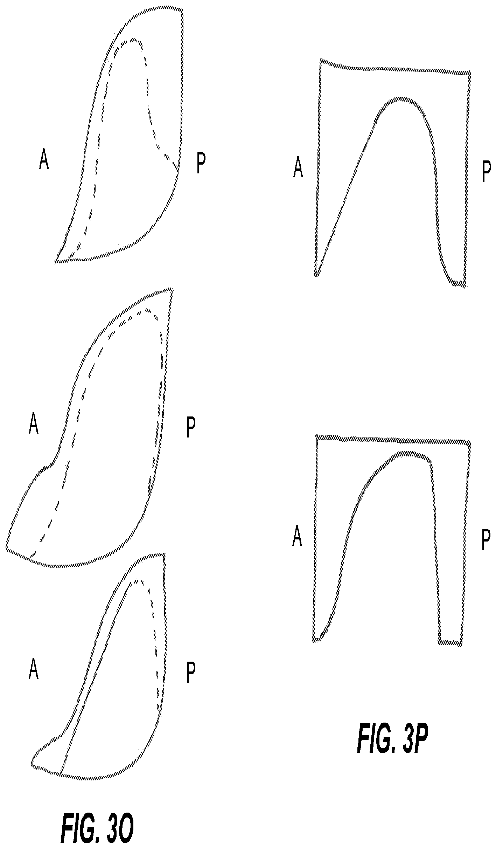

In various embodiments, an intercondylar box 4910 may be included in a femoral implant component, as shown, for example, in FIGS 1A and 1B. The intercondylar box can comprise a variety of configurations, shapes, and dimensions. For example, in some embodiments, the box 4910 can include a proximal wall 4912, which forms a "closed" configuration box. Alternatively, in some embodiments the box 4910 may not have a proximal wall 4912, and thus, comprise an "open" box configuration. Furthermore, in some embodiments, the box can include one or more planar surfaces that are substantially parallel or perpendicular to one or more anatomical or biomechanical axes or planes. Additionally or alternatively, in some embodiments, the box can include one or more planar surfaces that are oblique in one, two, or three dimensions. Similarly, in some embodiments, the box can include one or more curved surfaces that are curved in one, two, or three dimensions. FIGS. 3L through 3P depict anterior-posterior or lateral views of cross-sections of the internal surfaces of several different box embodiments. As shown, for example, in FIG. 3L, in some embodiments the internal surfaces of the box may be symmetrical, while in other embodiments the internal surfaces may be asymmetrical. As discussed further below, various aspects of the configuration, shape, and/or dimensions of the box may be standard or patient adapted.

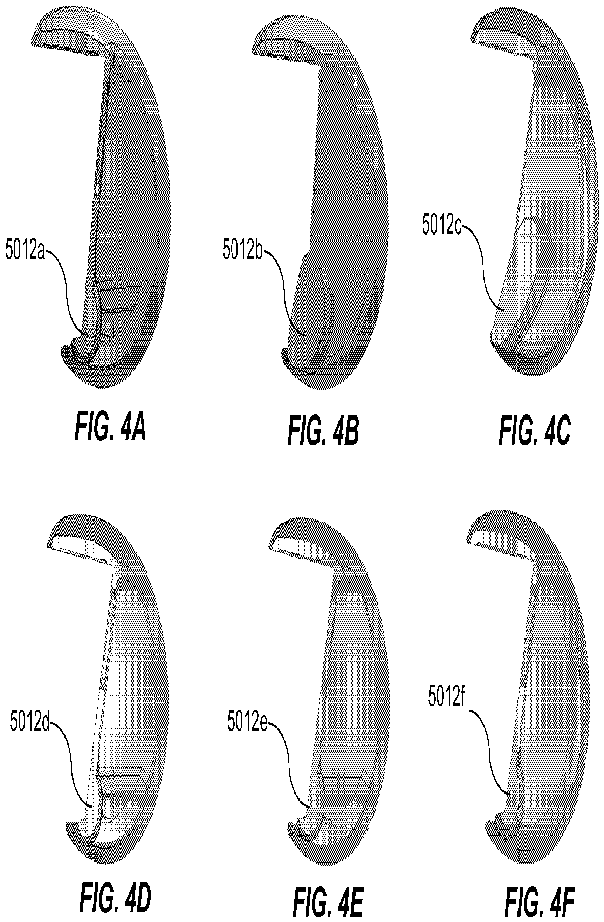

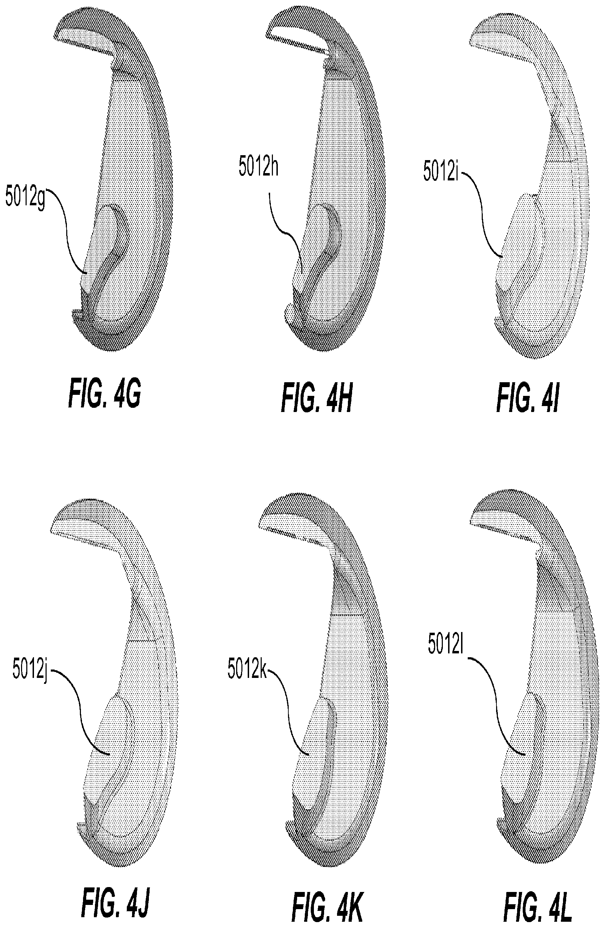

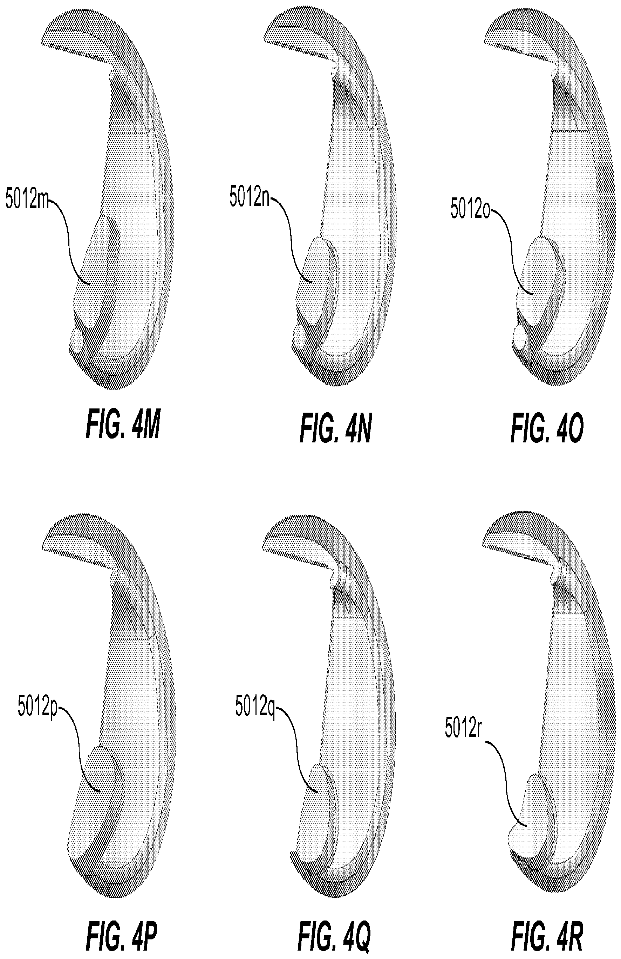

Additionally or alternatively, one or more intercondylar cams 5010, 5012 may be included in a femoral implant component, as shown, for example, in FIGS. 1B and 2B. Like the intercondylar box, intercondylar cams can comprise a variety of configurations, shapes, and dimensions. Some embodiments can include one cam, such as the femoral component shown in FIG. 1B, which includes only a posterior cam 5012. Other embodiments may include both an anterior cam 5010 and a posterior cam 5012. Some cams may be formed as independent structures extending between the medial and lateral condyle portions of the femoral implant, while other cams may be a portion of the femoral component forming a boundary of the intercondylar space, such as, for example, the edge of the femoral component forming the anterior boundary of the intercondylar space. In some embodiments, one or more cams may substantially comprise a cylindrical shape, including, for example, an elliptic, oblique, parabolic, or hyperbolic cylinder. In other embodiments, one or more cams may comprise irregular cross-sections, which can include one or more curvilinear and/or straight portions or sides. For example, FIGS. 4A-4R illustrate sagittal cross-sections of femoral components with exemplary cams 5012a-5012r. As shown, a femoral implant component can include a cam of a variety of shapes, sizes, and curvatures and one or more of these aspects can be patient adapted (i.e., patient-specific or patient-engineered). Exemplary methods of designing patient-adapted cams are described in detail below. Furthermore, while in some embodiments one or more cams may be symmetrical, one or more cams may also be asymmetrical. As discussed further below, various aspects of the configuration, shape, and/or dimensions of one or more cams may be standard or patient adapted.

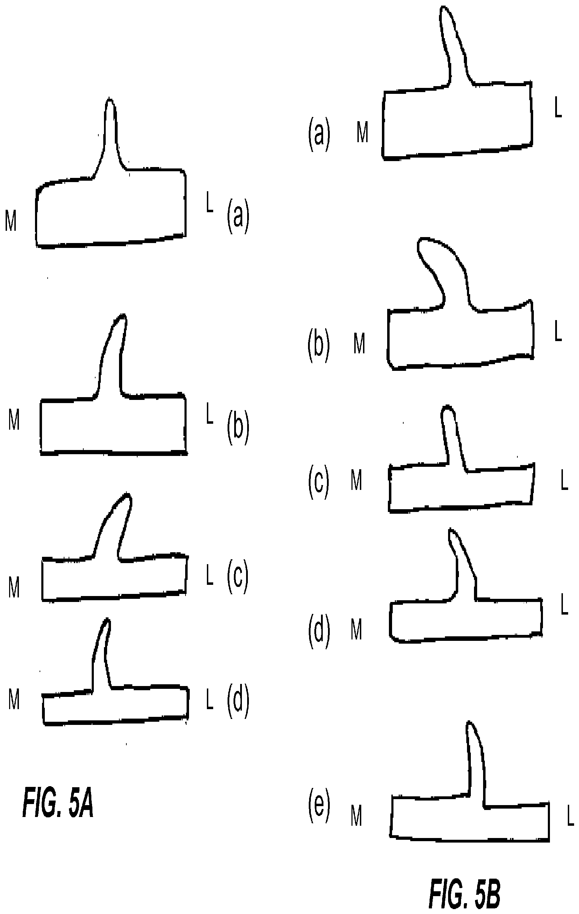

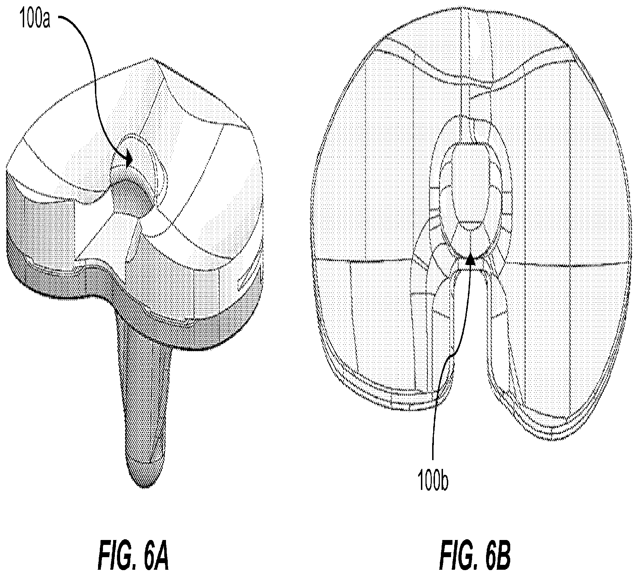

In some embodiments, a box and/or cam(s) of the femoral component may be configured to engage a post 5150 projecting from a tibial implant component (e.g., tibial tray, polyethylene insert). The post can comprise a variety of configurations, shapes, and dimensions. In some embodiments, the post may be substantially straight and perpendicular to the tibial plateau. Alternatively, the tibial post can have a curvature or obliquity in one or more dimensions, which can optionally be, at least in part, mirrored by a corresponding surface of the box and/or cam(s). FIGS. 5A and 5B depict cross-sections in a medial-lateral plane of exemplary post embodiments. FIG. 5A shows (a) a tibial implant component with a substantially straight post and (b)-(d) tibial implant components having posts oriented laterally, with varying thicknesses, lengths, and curvatures. FIG. 5B shows (a)-(e) posts oriented medially, with varying thicknesses, lengths, and curvatures. Various post embodiments similarly include at least portions oriented posteriorly or anteriorly, with varying thicknesses, lengths, and curvatures. For example, some post embodiments include a generally posterior-facing surface substantially angled and/or curved posteriorly as it extends from the tibial component. Additionally or alternatively, some post embodiments can include a generally anterior-facing surface, which may be substantially angled and/or curved posteriorly as it extends from the tibial component. In some embodiments, the post can have a substantially concave posterior-facing surface 100a, as illustrated in FIG. 6A, showing a perspective view of a tibial component. Alternatively, some embodiments can include a substantially convex posterior-facing surface 100b, as illustrated in FIG. 6B, showing a superior view of a tibial component. A substantially concave posterior-facing surface can help facilitate M-L rotation of the post relative to a cam and/or box (e.g., external rotation during flexion). The post can optionally taper or can have different diameters and cross-sectional profiles, e.g., round, elliptical, ovoid, square, rectangular, at different heights from its base. As discussed further below, various aspects of the configuration, shape, and/or dimensions of the post may be standard and/or patient adapted.

The tibial post may be designed to engage the box and/or cam(s) of the femoral component in various configurations. In embodiments with a box comprising a proximal wall 4912, the post may be configured to engage at least a portion of the distal-facing surface of the proximal wall 4912. For example, one or more surfaces of the post (including portions facing generally superiorly, anteriorly, and/or posteriorly) may be configured to engage and, optionally, pivot upon and/or translate across a portion of the distal-facing surface of the box's proximal wall 4912. In some embodiments, the distal-facing surface of the proximal wall 4912 may be sloped and/or curved in one or more dimensions. In some embodiments, the distal-facing surface of the proximal wall 4912 may include at least a portion that is patient-adapted, for example, as described below.

In addition to, or in place of, engagement with the box, in some embodiments, the post may be configured to engage one or more cams. For example, one or more surfaces of the post facing generally posteriorly (e.g., surfaces 100a and 100b), may be configured to engage a posterior cam of the femoral component. The posterior cam may be configured to pivot upon and/or translate (e.g., inferiorly, superiorly, medially, and/or laterally) across, at least a portion of, a generally posterior-facing surface of the post through at least a portion of flexion and/or extension. Additionally and/or alternatively, one or more surfaces of the post facing anteriorly and/or superiorly, may be configured to engage an anterior cam of the femoral component. In some embodiments, the anterior cam may be configured to pivot upon and/or translate (e.g., inferiorly, superiorly, medially, and/or laterally) across a generally anterior-facing surface of the post through at least a portion of flexion and/or extension.

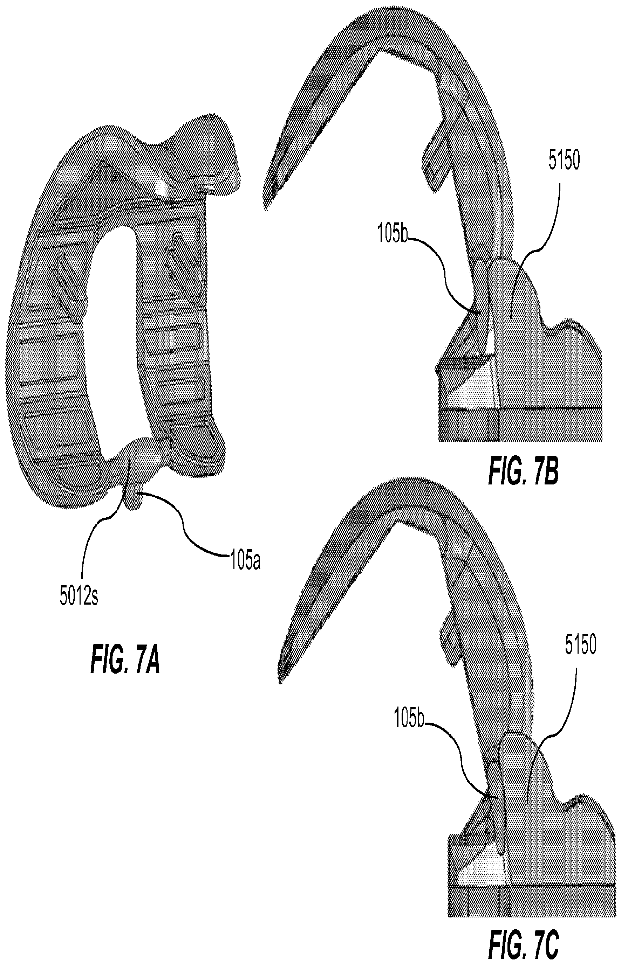

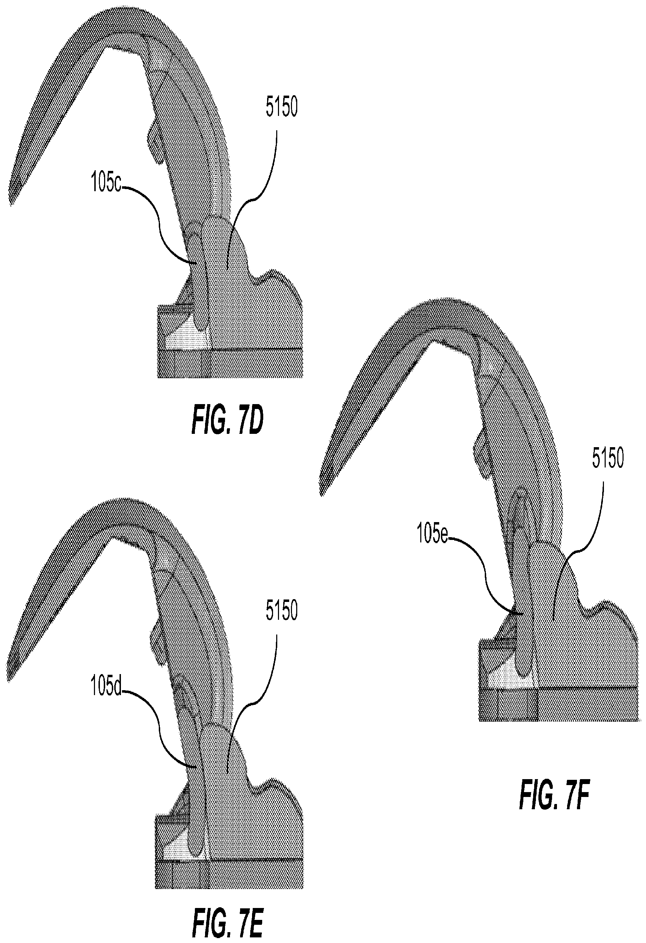

In some embodiments, one or more cams may further include a cam tongue (which may also be referred to herein as an "extension") extending from a portion of the cam, which may provide additional surface for engaging with the post through at least a portion of flexion and/or extension. For example, as shown in FIG. 7A, cam 5012s can include a cam tongue 105a extending generally posteriorly. Cam tongues can provide additional length and/or area of cam surface for engaging a post, which can, for example, functionally increase the jump-height of an implant configuration, facilitate cam-post engagement in deep flexion, and/or accommodate distribution of loading and forces between the cam and post over larger surface area(s). FIGS. 7B-7C illustrate sagittal cross-sections of an exemplary femoral component embodiment with a cam tongue 105b configured for engaging an exemplary tibial post 5150 at multiple angles of flexion (e.g., FIG. 7B, 7C). FIGS. 7D-7F illustrate sagittal cross-sections of additional femoral component embodiments with additional cam tongue configurations 105c-105e configured for engaging an exemplary tibial post 5150. As shown, a variety of configurations can be utilized and any one or more of the shape, size, and/or curvature of the cam, cam tongue, and/or post can be patient adapted (i.e., patient-specific or patient-engineered).

Additionally and/or alternatively, in some embodiments, the post can be configured to slide within a groove in a box and/or cam of the femoral implant. The groove may extend along a portion or substantially the entire anterior-posterior length of the box. In some embodiments, the groove can comprise stopping mechanisms at each end of the groove to prevent the post from dislocating from the track of the groove. The groove may have a width that extends across only a portion or substantially the entirety of the M-L box width. In some embodiments, the groove width may vary along the A-P length of the box.

In some embodiments, the post, box, and/or cam(s) may be configured to allow M-L rotation of the femoral component relative to the tibial component through at least a portion of flexion and/or extension. For example, in some embodiments, the cross-section of the portion of the post received by the box may be sufficiently smaller than the width of the box to allow M-L rotation of the post within the box. In some embodiments, the superior end of the post and/or a surface of the post that engages the box and/or cam(s) may be shaped to facilitate rotation and/or pivoting. For example, the superior end of the post and/or a surface of the post that engages the box and/or cam(s), or one or more portions thereof, may by substantially rounded, semi-spherical, or semicylindrical.

In some embodiments, the post, box, and/or cam(s) may be configured to guide and/or force M-L rotation of the femoral component relative to the tibial component through at least a portion of flexion and/or extension. For example, one or more surfaces of the post, box, and/or cam(s) may be sloped and/or curved (e.g., medially, laterally, anteriorly, posteriorly) over at least a portion of the surface that engages with the opposing post, box, and/or cam(s). By way of example, the anterior-facing and/or posterior-facing surfaces of the post may be sloped and/or curved so as to guide and/or force M-L rotation as that portion of the post engages, pivots upon, and/or translates across the box and/or cam(s). Similarly, the distal-facing surface of the proximal wall 4912 of the box and/or one or more cam surfaces may be sloped and/or curved so as to guide and/or force M-L rotation as the post engages, pivots upon, and/or translates across that box and/or cam surface.

Furthermore, in some embodiments, the slope and/or curvature of one or more surfaces of the post, box, and/or cam(s) may vary along one or more dimensions of the post, box, and/or cam(s). For example, an engagement surface's slope and/or curvature may vary (e.g., medially, laterally, anteriorly, posteriorly) along, at least a portion of, the length and/or width of the post, box, and/or cam(s). In some embodiments, this slope and/or curvature may increase in the direction along which the surface is engaged as flexion increases. For example, in some embodiments, a posterior cam may be configured to engage a posterior surface of a post, traversing the post in a generally inferior direction as flexion increases, and the post's posterior surface's slope and/or curvature with respect to an M-L axis may increase in the inferior direction, which can guide or force greater M-L rotation with greater flexion. Similarly, the slope and/or curvature with respect to an M-L axis of one or more surfaces of a cam may increase in the direction/order along which the one or more surfaces engage the post during flexion. In some embodiments, the slope and/or curvature of one engagement surface (e.g., the post's posterior surface) may substantially mirror the slope and/or curvature of the opposing engagement surface (e.g., the posterior cam surface that engages the post's posterior surface). As discussed further below, the slope and/or curvature of one or more surfaces of the post, box, and/or cam(s) may be standard or patient adapted.

In various embodiments, the post, box, and/or cam(s) can include features that are patient-adapted (e.g., patient-specific or patient-engineered). For example, one or more of the configurations, shapes, dimensions, slopes, curvatures, and/or positions of the post, box, and/or cams may be patient-adapted. Accordingly, one or more features of posterior-stabilizing implant components of various embodiments herein can be designed and/or selected, based, at least in part, on patient-specific data, including, for example, one or more of: intercondylar distance or depth; femoral shape; condyle shape; lateral and/or medial tibial plateau slope, convexity/concavity, A-P length, M-L length, offset; lateral and/or medial tibial spine locations; ACL, PCL, MCL, and/or LCL origin location, insertion location, orientation, or physical or force direction; and one or more of the parameters listed in Table 3 and/or Table 4 below. Additionally or alternatively, additional patient characteristics can also be utilized, including, for example, weight, height, sex, bone size, body mass index, muscle mass; and/or any other patient-specific information described herein. By way of example, in some embodiments, one or more dimensions of the post, box, and/or cam(s) can be designed and/or selected to avoid patellar surface impingement. Alternatively or in addition, one or more features of the post, box, and/or cam(s) can be engineered based on patient-specific data and, optionally, additional data, such as, for example, implant component material properties and/or desired kinematic properties (obtained from, e.g., population database, biomotion modeling, clinical studies). For example, the dimensions of the post, box, and/or cam(s) can be designed and/or selected based on a minimum allowable thickness determined based on one or more of the material properties of the post, box, and/or cam(s) and the patient's weight, height, sex, bone size, body mass index, and/or muscle mass.

Accordingly, in some embodiments, various dimensions of the post, box and/or cam(s) can be designed and/or selected based, at least in part, on various patient dimensions and/or implant dimensions. Examples of embodiments are provided in Table 1. These examples are in no way meant to be limiting.

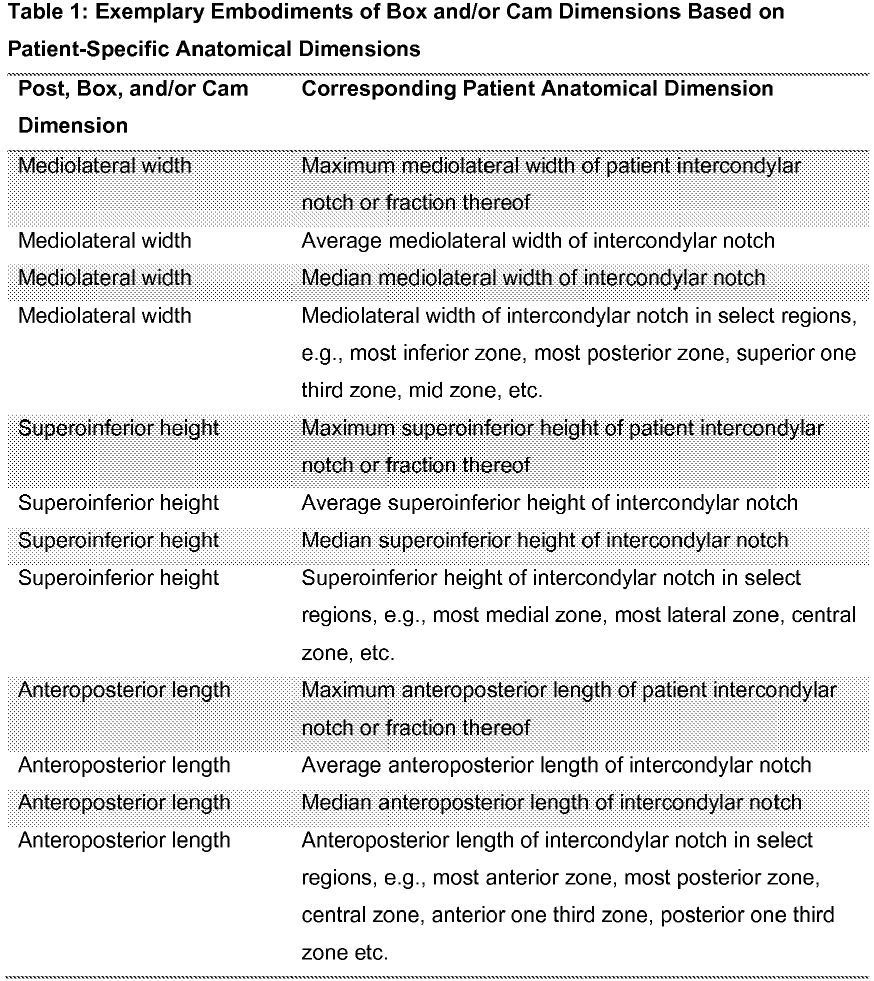

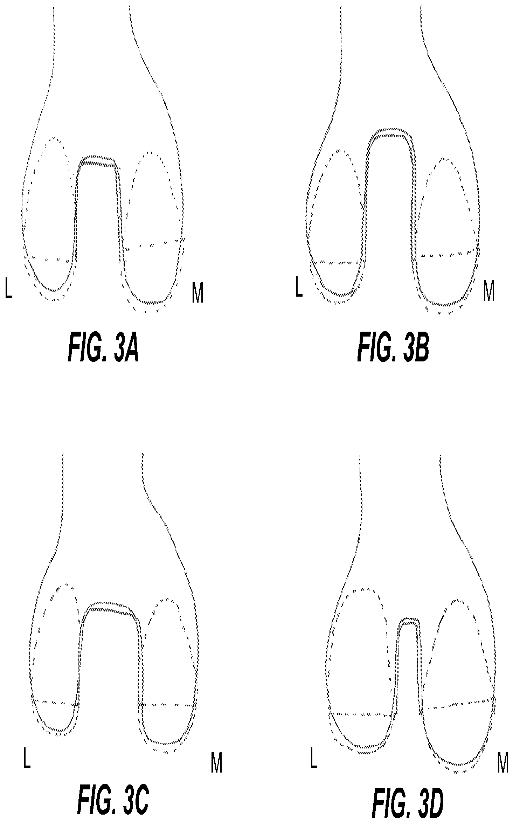

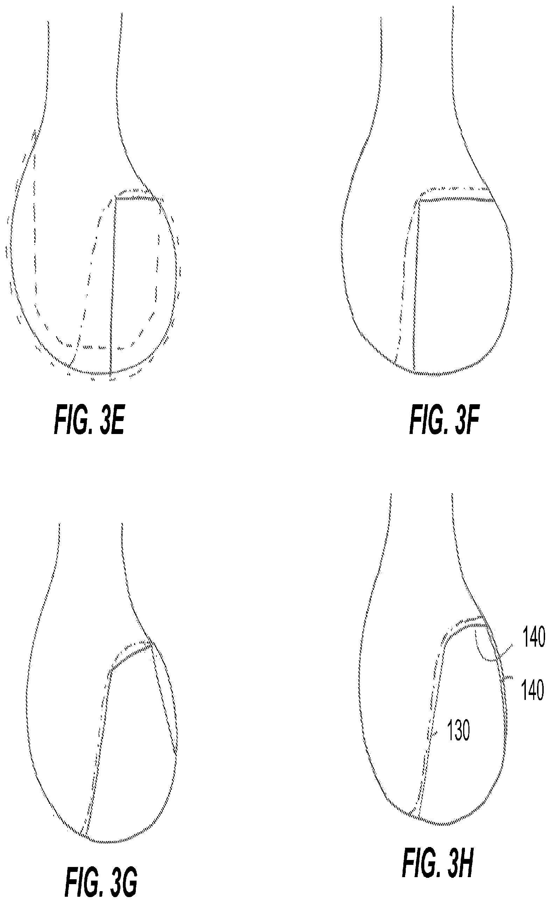

FIGS. 3A through 3P show various exemplary embodiments of an intercondylar box. FIG. 3A shows a box height adapted to the superoinferior height of the intercondylar notch. The dotted outlines indicate portions of the bearing surface and posterior condylar surface as well as the distal cut of the implant. FIG. 3B shows a design in which a higher intercondylar notch space is filled with a higher box, for example, for a wide intercondylar notch. FIG. 3C shows a design in which a wide intercondylar notch is filled with a wide box. The mediolateral width of the box is selected and/or designed based on the wide intercondylar notch. FIG. 3D shows an example of an implant component having a box designed for a narrow intercondylar notch. The mediolateral width of the box is selected and/or designed for the narrow intercondylar notch. FIG. 3E shows an example of an implant component having a box for a normal size intercondylar notch. The box is selected and/or designed for its dimensions. (Notch outline: dashed and stippled line; implant outline: dashed lines). FIG. 3F shows an example of an implant component for a long intercondylar notch. The box is designed, adapted or selected for its dimensions (only box, not entire implant shown).

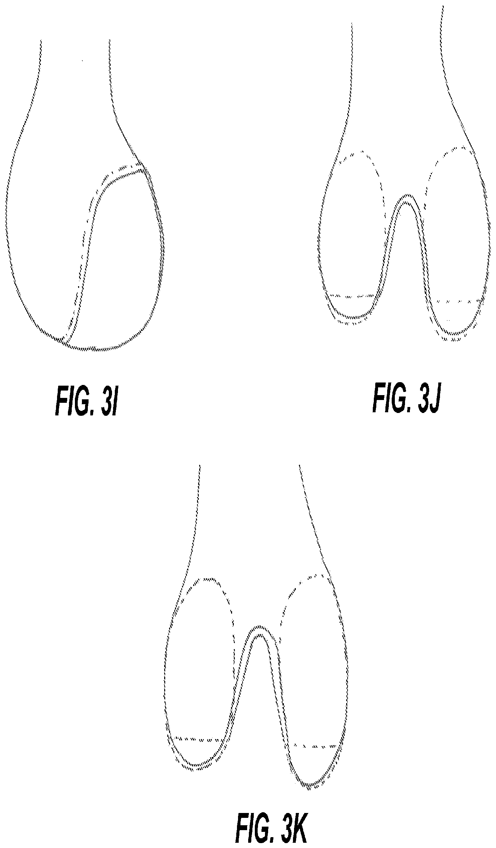

FIG. 3G is an example of one or more oblique walls that the box can have in order to improve the fit to the intercondylar notch. FIG. 3H is an example of a combination of curved and oblique walls that the box can have in order to improve the fit to the intercondylar notch. FIG. 3I is an example of a curved box design in the A-P direction in order to improve the fit to the intercondylar notch. FIG. 3J is an example of a curved design in the M-L direction that the box can have in order to improve the fit to the intercondylar notch. Curved designs are possible in any desired direction and in combination with any planar or oblique planar surfaces. FIG. 3K is an example of oblique and curved surfaces in order to improve the fit to the intercondylar notch. Alternatively or additionally, the box can form an opening having a generally longitudinal axis extending at an angle relative to a sagittal plane. In some such embodiments, either or both of the medial and lateral walls (including one or more bone-facing surfaces and/or one or more intercondylar facing surfaces) of the box may be angled relative to a sagittal plane. In some embodiments, one or more of such angles relative to a sagittal plane may be based on patient-specific information, including, for example, any of the parameters listed in Tables 3 and 4 below.

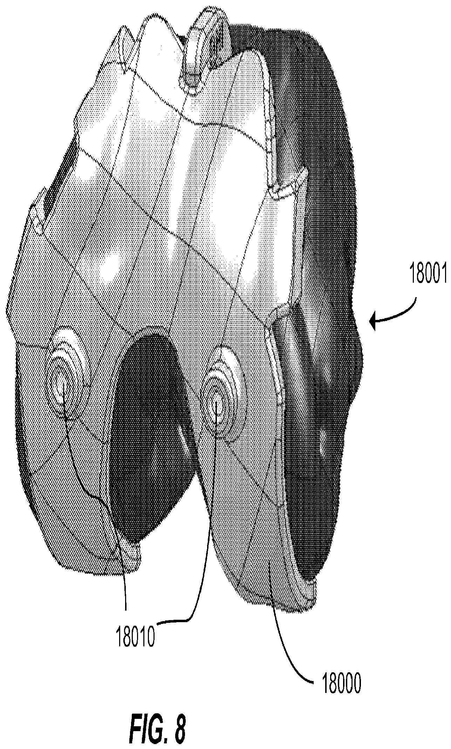

In various embodiments, preparation of an implantation site for a posterior stabilizing implant can include the use of one or more patient-adapted surgical techniques, cutting guides, and/or instruments. Such surgical techniques, cutting guides, and/or instruments can include, for example, any of those described in U.S. Patent Publication No. 2012-0209394, including those discussed for use in non-posterior-stabilizing techniques (e.g., cruciate retaining techniques). For example, as an initial step in guiding a surgeon for preparation of the femur for the implantation of a patient-adapted femoral implant, a femoral jig 18000, as illustrated in FIG 8. can be used to, for example, align and locate guide pins (i.e., Steinman Pins) for placing various jigs used for aligning subsequent femoral cuts. This jig 18000 can incorporate an inner surface (not shown) that substantially conforms to some or all of the outer surface of the uncut distal femur 18001 (e.g., cartilage and/or subchondral bone), whereby the jig fits onto the femur in desirably only one position and orientation. In various embodiments, the jig 18000 can comprise a flexible material which allows the jig 18000 to flex and "snap fit" around the distal femur. In addition, the inner surface of the jig can be intentionally designed to avoid and/or accommodate the presence of osteophytes and other anatomical structures on the femur 18001. A pair of pin openings 18010, extending through the surface of the jig, can provide position and orientation guidance for a pair of guide pins that can be inserted into the distal surface of the femur (not shown). The jig 18000 can then be removed from the femur 18001 and subsequent steps for preparing the femur can be performed (e.g., placement of one or more bone cuts corresponding to the bone-facing surfaces of the patient-adapted femoral implant), optionally, utilizing and/or referencing the position and/or orientation of the guide pins.

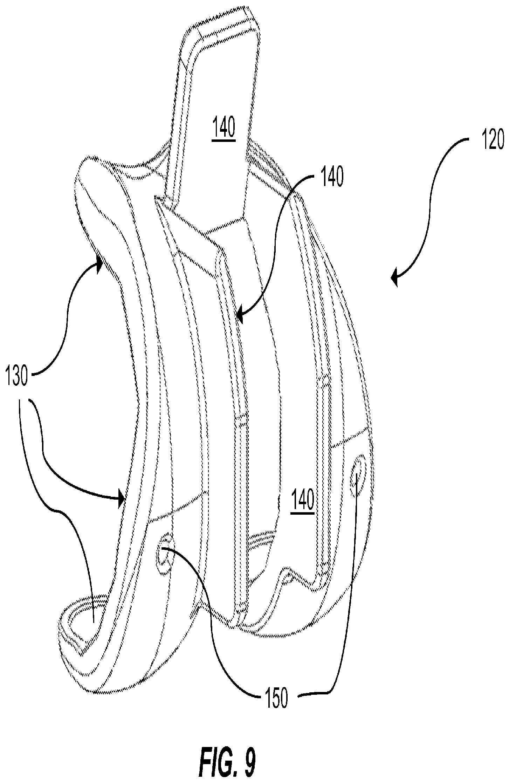

Additionally or alternatively, some embodiments may include the use of, for example, a patient-adapted cutting guide configured for guiding one or more femoral box cuts. For example, some embodiments can include a femoral box-cut guide 120 as depicted in FIG. 9. Such a cutting guide may be derived, generally, for example, from the design for a patient-adapted femoral implant. Accordingly, in some embodiments, one or more bone-facing surfaces 30 of the cut guide may be configured to engage one or more bone cuts planned for the femoral implant. In some embodiments, one or more bone facing surfaces of the femoral box-cut guide may be configured to engage uncut bone and/or cartilage, based, for example, on patient-specific information. The femoral box-cut guide 120 can include one or more box-cut guide surfaces 140. One or more of the box-cut guide surfaces 140 can include one or more features (e.g., position, shape, size, curvature, slope) based, at least in part, on patient-specific information. Additionally, in some embodiments, a femoral box-cut guide 120 can include one or more pin holes 150, which may be patient adapted, and which may facilitate stabilization of the guide and/or referencing other cutting guides and/or drilling instruments.

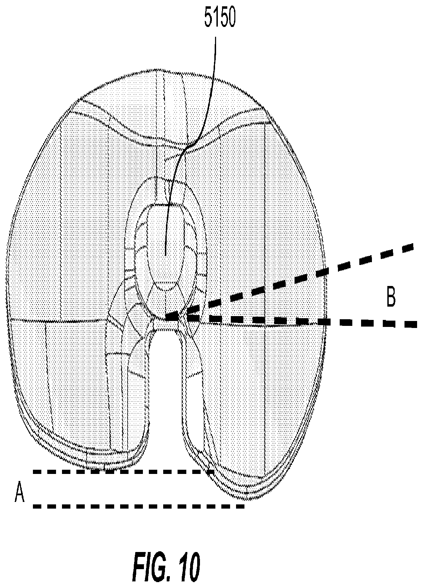

As discussed above, in various embodiments, the length, width, height, orientation, slope and/or curvature of one or more portions of the post, box, and/or cam(s) can be designed and/or selected to be patient-adapted based on patient-specific information. In some embodiments, one or more shapes and/or curvatures of at least a portion of the post may be patient-adapted. For example, in some embodiments, a position and/or curvature of the post may be designed to allow and/or guide a desired amount of external rotation and/or posterior-lateral rollback based on a difference in anterior-posterior dimension between the medial and lateral compartments, for example, as depicted in FIG. 10. For example, a degree of angular shift B of post 5150a may be determined based on the length difference A between the medial and lateral compartments.

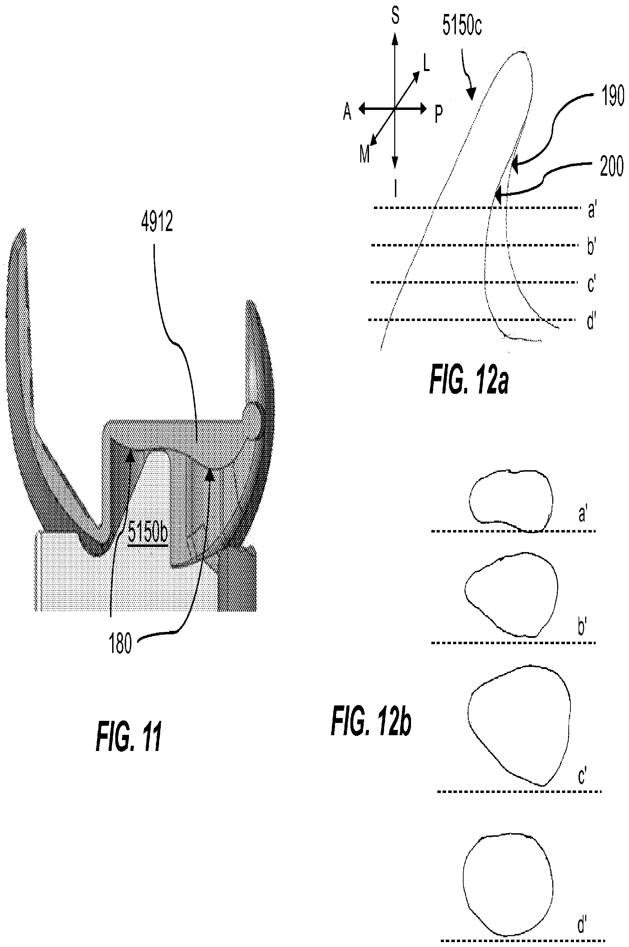

In some embodiments, one or more features of the post, box, and/or cam(s) may be based on at least a portion of one or more patient-specific femoral sagittal curvatures, lines, and/or angles (e.g., trochlear J-curve, medial condylar J-curve, lateral condylar J-curve, Blumensaat line, and/or curvature of the roof of the intercondylar notch) derived, for example, as disclosed in U.S. Patent Publication No. 2012-0209394. For example, Fig. 11 depicts a sagittal cross-sectional view of an embodiment in which the distal-facing surface 180 of the box proximal wall 4912 includes a sagittal curvature correlated to the changing centers of curvature of the femoral condyles. Similarly, in some embodiments, one or more edges and/or surfaces of the post may be selected and/or designed based on at least a portion (e.g., anterior, distal, proximal, or combinations and/or portions thereof) of one or more femoral sagittal curvatures, lines, and/or angles. For example, Fig. 12a depicts an exemplary embodiment of a post 5150c, having a lateral posterior edge portion 190 and a medial posterior edge portion 200. The shape and/or curvature of one or both of the medial and lateral posterior edge portions 190, 200 can be based on one more patient-specific sagittal curvatures, lines, and/or angles (or portions thereof). For example, a shape of a lateral posterior edge portion 190 can based on a posterior portion of a lateral femoral J-curve, while a shape of medial posterior edge portion 200 can be based on a posterior portion of a medial femoral J-curve. Accordingly, such a patient-specific post can have varying cross-sections, for example, as illustrated in FIG. 12b, showing transverse cross-sections of post 5150c relative to lines a', b', c', and d' in FIG. 12a. Note, in various embodiments, the edge portions of the post referred to herein may comprise substantially sharp edges and/or substantially curved, chamfered, or rounded edges.

As mentioned above, in some embodiments, one or more features of the post, box, and/or cam(s) may be based on a portion (e.g., anterior, distal, proximal, or combinations and/or portions thereof) of one or more femoral sagittal curvatures, lines, and/or angles. For example, in some embodiments, a post curvature may be based on a portion of a posterior femoral J-curve. Additionally and/or alternatively, in some embodiments, particular portion(s) and/or relative angle(s) of the one or more curvatures, lines, and/or angles, can be determined based, for example, on its location and/or orientation during one or more portions of flexion, extension, and/or engagement of the post and box/cam. For example, in some embodiments, the portion of a condylar J-curve contacting a tibial surface at the same time contact first occurs between the cam and post may be used to derive a feature of the post, box, and/or cam(s). Similarly, in some embodiments, a shape and/or position of a post and/or cam can be determined based on the angle of the Blumensaat line relative to an anatomical axis at varying degrees of flexion, extension, and/or engagement of the post and box/cam. A shape and/or position of multiple positions of the post and box/cam can each be based on the particular angle of the Blumensaat line relative to an anatomical axis when desired and/or modeled engagement occurs between the post and box/cam at the respective positions.

Furthermore, as will be appreciated, the particular relationship between one of the exemplary patient-specific parameters discussed above and a posterior-stabilizing feature can comprises a variety of forms in addition to direct matching. For example, in some embodiments, a mathematical function (e.g., linear, non-linear) may be used to correlate a patient-specific anatomical curvature to a post, box, and/or cam curvature. Additionally or alternatively, in some embodiments, simulations (e.g., kinematic and/or non-kinematic modeling) may be used derive a relationship to be used for selecting and/or designing a given posterior stabilizing feature based on patient-specific parameters.

In some embodiments, for example, a simulation can begin with modeling articular surfaces of femoral and/or tibial components, based, for example, on a cruciate-retaining design (e.g., as disclosed in U.S. Patent Publication No. 2012-0209394). Next, varying predictiles can be created by modeling the components in engagement at varying degrees of flexion/extension. Relative locations of features (e.g., length, width, height, orientation, slope and/or curvature) of portions of a proposed box, post, and/or cam(s) can be determined based on one or more of the predictiles. Optionally, additional standard and/or patient-specific parameters may also be utilized in such simulations. For example, in some embodiments, a desired angle of flexion at which post and box/cam engagement begins can be set (e.g., at about 10, about 20, about 30, about 40, about 50, about 60, or about 70 degrees of flexion). Additionally or alternatively, a desired maximum flexion angle may also be set (e.g., at about 130, about 135, about 140, about 145, about 150, about 155, or about 160 degrees of flexion). These exemplary flexion angle parameters may be patient specific or standard in various embodiments.

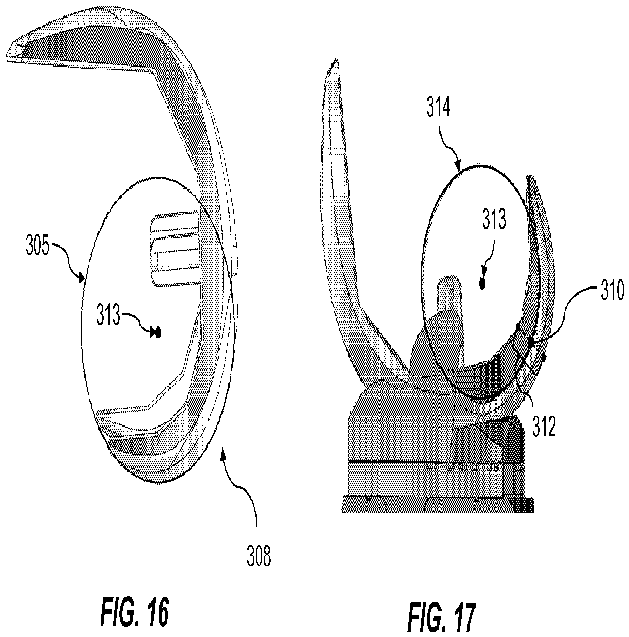

In certain embodiments, a cam (optionally, including a tongue), or portion thereof, may be selected and/or designed based on modeling engagement of a femoral implant component (e.g., any of the patient-adapted femoral implant components disclosed herein) and a tibial implant component that includes a post (standard or patient-adapted) through one or more degrees of flexion and/or extension. For example, in some embodiments, a cam may be centered on or about the mid-thickness of the condyle. This may be determined by deriving a circle 305 best-fit to a portion (e.g., posterior) of the sagittal curvature 308 of the implant condyle shape, as illustrated in FIG. 16. The midpoint 310 of the condylar thickness 312 may be determined at one of the chamfer corners, as shown in FIG. 17, and a circle 314 may then be created using the center point 313 of circle 305 and extending out to midpoint 310. Next, in some embodiments, the femoral component may be positioned with its sagittal plane aligned with the tibial component's sagittal plane and at a starting flexion angle (e.g., at about 10, about 20, about 30, about 40, about 50, about 60, or about 70 degrees of flexion) for modeling.

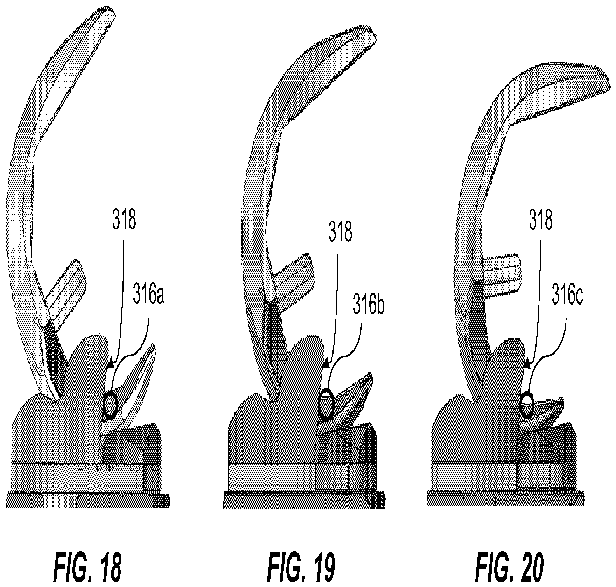

In some modeling embodiments, the initial flexion angle may be set at about 60°. Additionally, in some embodiments, the components may be further positioned such that a particular femoral bearing point (e.g., inferior-most point of condylar surface, i.e., joint-facing surface, at the given flexion angle) is aligned with a particular tibial bearing point (e.g., inferior-most point of a tibial articulating surface, i.e., joint-facing surface). With the component positions set, a circle 316a may be derived that is tangent to a cam bearing surface 316a and centered on circle 314, as illustrated in FIG. 18. Then, in some embodiments, the sagittal planes of the femoral and tibial components may be realigned, if needed, the flexion angle may be adjusted, and the steps described above may be repeated one or more times at varying angles of flexion to derive corresponding circles 316. For example, FIG. 19 illustrates a circle 316b derived at 75° and FIG. 20 illustrates a circle 316c derived at 90°.

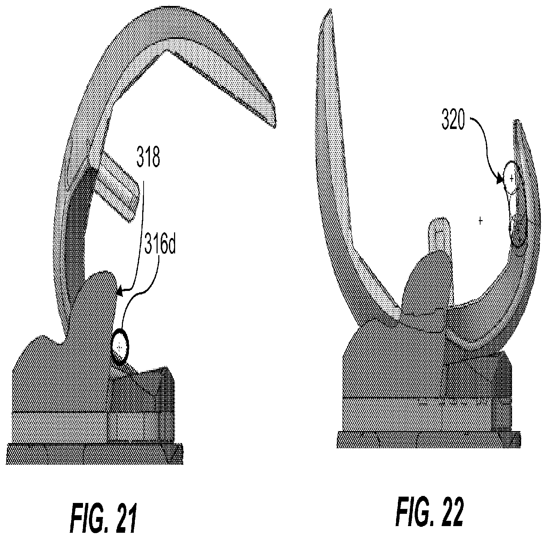

Optionally, in some embodiments, at one or more flexion angles in the above method, the relative positions of the particular femoral bearing point and particular tibial bearing point may be adjusted (e.g., to account for femoral rollback on the tibia). For example, in FIG. 21, the flexion angle is set at 120°, and the particular femoral bearing point (e.g., inferior-most point of condylar surface at the given flexion angle) is positioned 3mm posterior to the particular tibial bearing point (e.g., inferior-most point of a tibial articulating surface). Then, as described above, a circle 316d may be derived. The flexion angles at which relative positions of the bearing points are adjusted, as well as the amount and direction may be based on a variety of factors, including, for example, any of the patients-specific parameters described herein (e.g., in Tables 3 and 4) and/or generalized information regarding joint kinematics. For example, in some embodiments, one or more of the flexion angles at which relative positions of the bearing points are adjusted, the amount of adjustment, and the direction of adjustment may be derived on generalized kinematic information correlating femoral rollback and/or femoral rotation to one or more patient-specific parameters (e.g., height, weight, formal width).

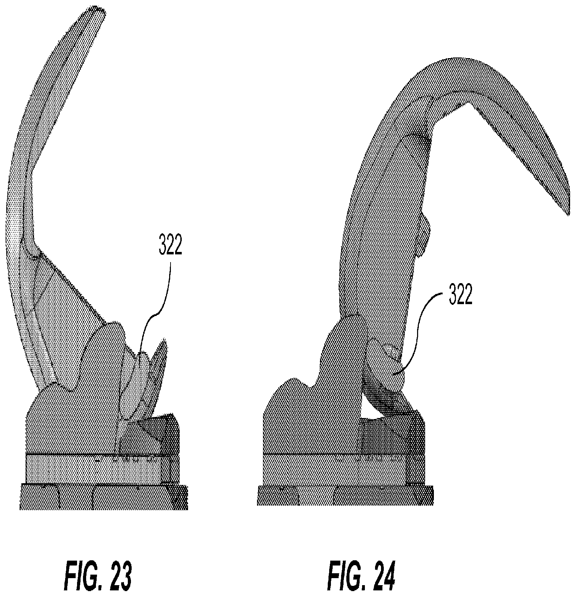

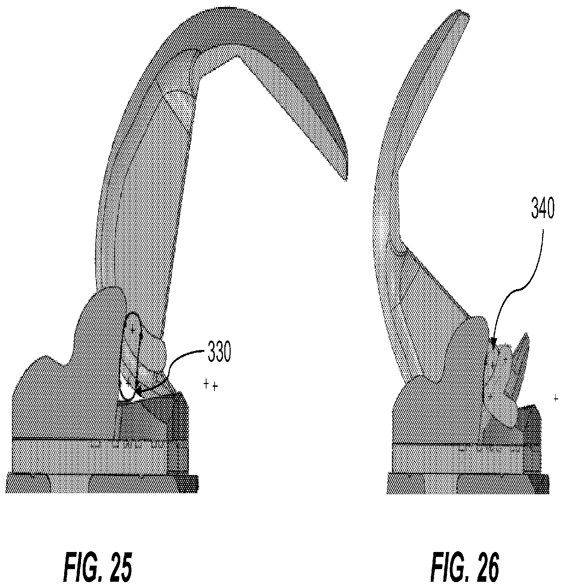

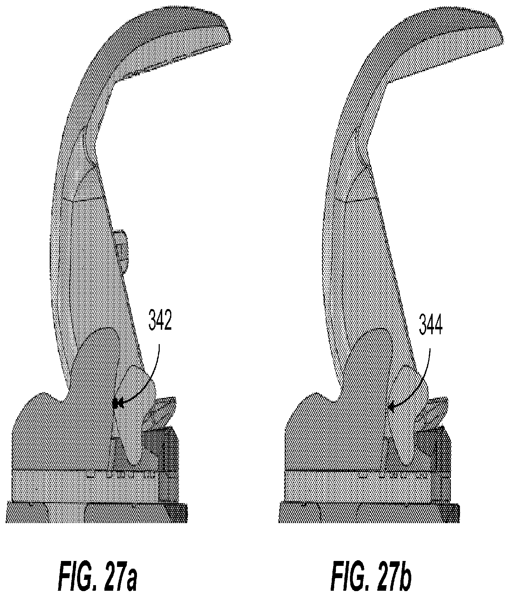

Furthermore, in some embodiments of the modeling methods above, each of the derived circles 316a-d may be mapped into one view and an arc 320 may be created using the circles 316a-d as a guide for a peripheral arc of curvature, as shown in FIG. 22. FIG. 23 shows resultant cam extrusion 322 at 60° and FIG. 24 shows the cam extrusion 322 at 120°. Optionally, additional modifications can be made to the cam 322 to, for example, increase jump-height and/or optimize point loading and/or surface forces between the cam and post. For example, Fig. 25 illustrates extending 330 the cam to maximize the contact area at 120° of flexion. Similarly, Fig. 26 illustrates extending 340 the cam to maximize the contact area at 60° of flexion. Fig. 27a illustrates identifying an angle at which contact area 342 is at the lowest, and Fig. 27b illustrates modifying the cam by rounding 344 the cam surface around point 342 to optimize the contact area.

Additionally or alternatively, one or more features of the post, box, and/or cam(s) may be based on patient-specific and/or desired kinematic properties, including, for example, M-L rotation, femoral rollback, and/or any one or more of the other exemplary parameters listing in Table 3 below. For example, as discussed above, in some embodiments, one or more surfaces of the post, box, and/or cam(s) may be sloped and/or curved (e.g., medially, laterally, anteriorly, posteriorly) over at least a portion of the surface that engages with the opposing post, box, and/or cam(s) in order to guide and/or force M-L rotation (e.g., femoral external rotation) of the femoral component relative to the tibial component and femoral rollback (e.g., lateral femoral rollback). Accordingly, in some embodiments, the nature and degree of the slope and/or curvature of the one or more surfaces of the post, box, and/or cam(s) may be based on a patient-specific and/or desired M-L rotation and rollback.

In various embodiments, patient-specific ligament (e.g., ACL, PCL, MCL, LCL) information (e.g., origin location, insertion location, orientation, physical or force direction), may be used to select and/or design posterior stabilizing features. In some embodiments, such ligament information may be derived from kinematic information (e.g., from measured patient-specific information or from modeling based on average kinematics for a particular relevant population group). Additionally or alternatively, in some embodiments, such ligament information may be obtained from bony landmarks (e.g., based on directly measured patient-specific locations or based on locations derived from information correlating average locations to other measureable patient-specific information). Optionally, in certain embodiments, such ligament information may also be obtained directly from soft-tissue imaging of the patient.

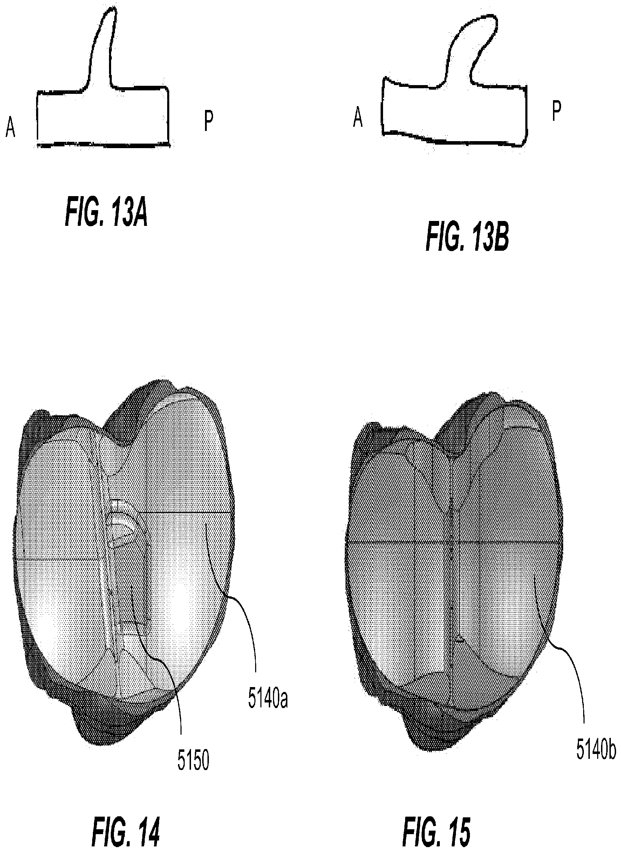

In some embodiments, the post can slope and/or curve medially, laterally, anteriorly, and/or posteriorly as it extends from its base to its tip, as discussed above and as depicted, for example, in FIGS. 5A and 5B. The anterior surface of the post, posterior surface of the post, or both may be patient-adapted. For example, the M-L and/or A-P slope and/or curve of the anterior and/or posterior surface of the post can be patient-derived or patient-matched (e.g., to match the physical or force direction of the PCL or ACL). Further, in some embodiments, the sagittal curve of one or more surfaces of the post can be based on the PCL location and orientation or combinations of ACL and PCL location and orientation. In some embodiments, the shape of one or more surfaces of the post may be patient-adapted (in, e.g., the sagittal plane) to optimize rollback for the particular patient. Desired rollback may be modeled based on, for example, the dimensions of the patient's tibial plateau, e.g., A-P dimension and/or M-L dimension, oblique dimension, and/or combinations thereof. In some embodiments, one or more sagittal dimensions, slopes, and/or curvatures of the post may be based on and/or proportional to an A-P length (e.g., average A-P length) of the patient's tibial plateau. For example, the post depicted in FIG. 13A may be appropriate for a patient with a relatively smaller tibial plateau A-P length, while the post depicted in FIG. 13B, extending further posteriorly, may be appropriate for a patient with a relatively larger tibial plateau A-P length.

Further examples of patient dimensions and/or implant dimensions upon which corresponding post dimensions can be based, at least in part, in some embodiments are provided in Table 2. These examples are in no way meant to be limiting.

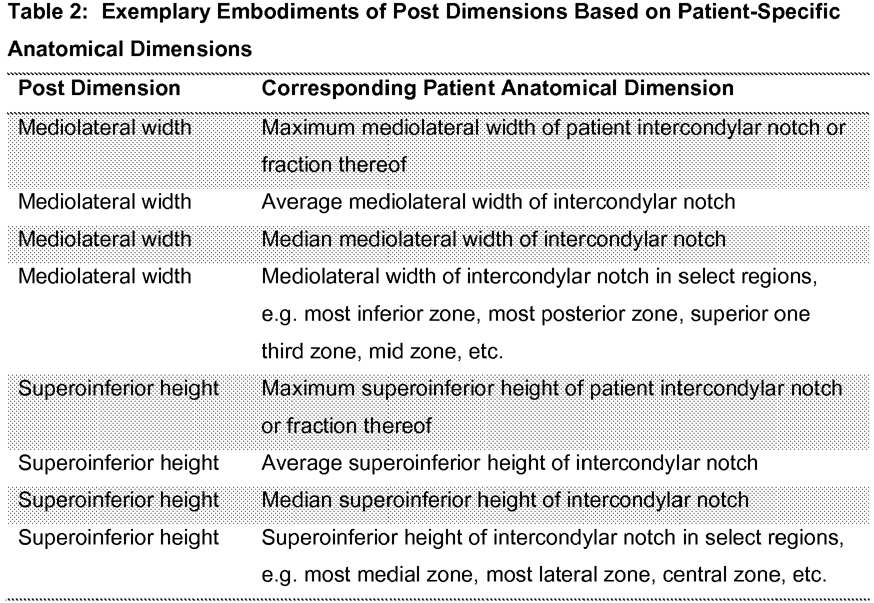

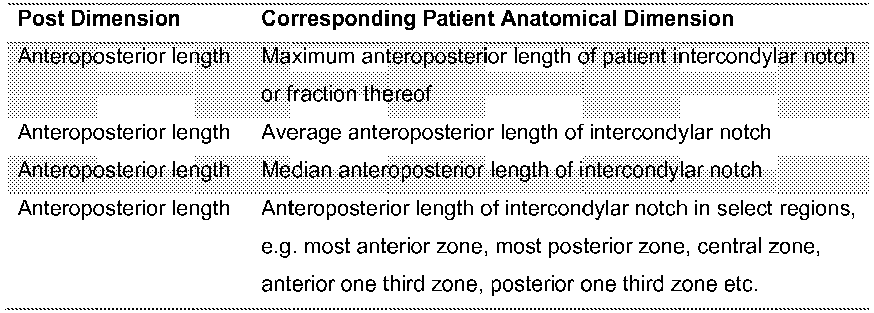

In some embodiments, the position of the post can be adapted based on patient-specific dimensions. For example, the post can be matched with or adapted relative to or selected based on the position or orientation of the ACL or the PCL origin and/or insertion. Alternatively, the post can be placed at a predefined distance from the ACL and/or PCL insertion, from the medial or lateral tibial spines, or from other bony or cartilaginous landmarks or sites. The position of the post can be matched with or adapted relative to or selected based on anatomical dimensions or landmarks, such as, for example, a femoral condyle shape, a notch shape, a notch width, a femoral condyle dimension, a notch dimension, a tibial spine shape, a tibial spine dimension, a tibial plateau dimension, and/or an ACL, PCL, MCL, and/or LCL origin or insertion location.

Similarly, the position of the box and/or cam(s) on the femoral component can be designed, adapted, or selected to be close to the PCL origin or insertion or at a predetermined distance to the PCL or ACL origin or insertion or other bony or anatomical landmark. The position of the box and/or cam(s) can be matched with or adapted relative to or selected based on anatomical landmarks or dimensions, e.g., a femoral condyle shape, a notch shape, a notch width, a femoral condyle dimension, a notch dimension, a tibial spine shape, a tibial spine dimension, a tibial plateau dimension, and/or an ACL, PCL, MCL, and/or LCL origin or insertion location.

In addition to the various patient-adapted configurations and corresponding parameters described above, one or more features of the post, box, and/or cam(s) may be adapted based on additional parameters, such as, for example, those discussed and listed below in Table 4 and/or parameters obtained through patient-specific and/or generalized biomotion models. For example, in some embodiments, the length, width, height, orientation, slope, curvature, and/or position of the post, box, and/or cam(s) may be selected and/or designed based on one or more of the exemplary parameters listed in Table 3. These examples are in no way meant to be limiting.

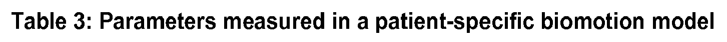

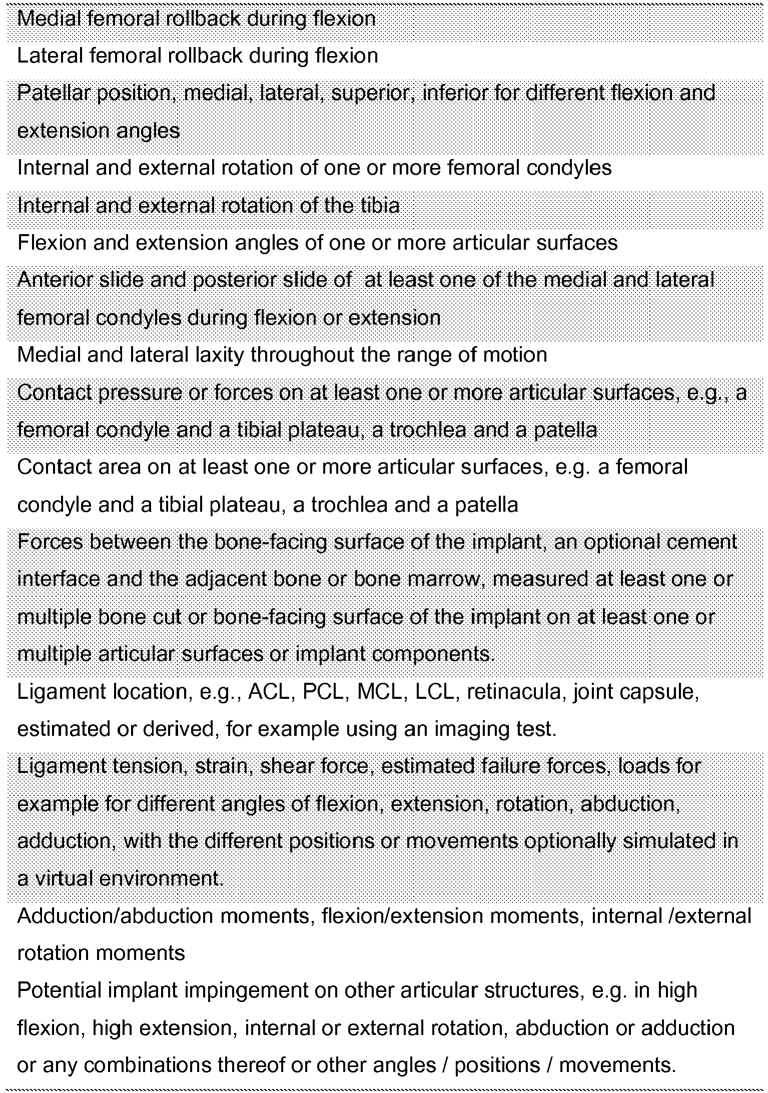

Additionally or alternatively, in some embodiments, the dimensions of the post, box, and/or cam(s) can be selected and/or designed based, at least in part, on the intended implantation technique, or properties thereof, such as, for example intended flexion, rotation, and/or tibial slope. For example, at least one of an anteroposterior length or superoinferior height can be adjusted if an implant is intended to be implanted in 7 degrees flexion as compared to 0 degrees flexion, reflecting the relative change in patient or trochlear or intercondylar notch or femoral geometry when the femoral component is implanted in flexion.

In another example, the M-L width can be adjusted if an implant is intended to be implanted in internal or external rotation, reflecting, for example, an effective elongation of the intercondylar dimensions when a rotated implantation approach is chosen. The post, box, and/or cam(s) can include oblique or curved surfaces, typically reflecting an obliquity or curvature of the patient's anatomy. For example, the superior portion of the box and/or cam(s) can be curved reflecting the curvature of the intercondylar roof. In another example, at least one side wall of the box can be oblique reflecting an obliquity of one or more condylar walls.

The posterior stabilizing features described above may be integrally formed with other components of the articular repair system or may be modular. For example, in certain embodiments, the femoral implant component can be designed and manufactured to include a box and/or cam as a permanently integrated feature of the implant component. Alternatively, in certain embodiments, a box and/or cam can be modular. For example, the box and/or cam can be designed and/or manufactured separate from the femoral implant component and optionally joined with the component, either prior to (e.g., preoperatively) or during the implant procedure. Methods for joining a modular box to an implant component are described in the art, for example, in U.S. Patent No. 4,950,298. In some embodiments disclosed herein, modular cams can be joined to an implant component at the option of the surgeon or practitioner, for example, using spring-loaded pins at one or both ends of the modular cams. The spring-loaded pins can slideably engage corresponding holes or depressions in the femoral implant component.

Similarly, in certain embodiments, a tibial implant component can be designed and manufactured to include a post as a permanently integrated feature of the implant component. Alternatively, in some embodiments, the post can be modular. For example, the post can be designed and/or manufactured separate from the tibial implant component and optionally joined with the component, either prior to (e.g., preoperatively) or during the implant procedure. For example, a modular post and a tibial implant component can be mated using an integrating mechanism such as respective male and female screw threads, other male-type and female-type locking mechanisms, or other mechanisms capable of integrating the post into or onto the tibial implant component and providing stability to the post during normal wear. A modular post can be joined to a tibial implant component at the option of the surgeon or practitioner, for example, by removing a plug or other device that covers the integrating mechanism and attaching the modular post at the uncovered integrating mechanism. In some embodiments, a surgical kit may include a plurality of different posts configurations (standard and/or patient-adapted) from which the surgeon can select.

In some embodiments, the tibial implant component that the post is integral with, or configured to be joined to, may be a tibial tray. For example, the post may project from a joint facing surface of a tibial tray. Accordingly, one or more polyethylene inserts may be configured to wrap around the tibial post when inserted into the tibial tray. For example, in some embodiments in which medial and lateral polyethylene inserts are to be positioned on the tibial tray, the medial insert, the lateral insert, or both may include a cutout along a mesial edge to accommodate the tibial post.

In other embodiments, the tibial implant component that the post is integral with, or configured to be joined to, may be a polyethylene insert configured to be disposed on a tibial tray. In tibial implant embodiments comprising a medial and lateral polyethylene insert, the post may be configured to project from the medial insert, the lateral insert, or both. In some embodiments, it may be desirable to alter the size and shape of the medial and lateral polyethylene inserts relative to what their size and shape would be in a tibial implant not configured for posterior stabilization. For example, in some embodiments, the mesial edge of a medial insert 5140 may extend further laterally and may extend posteriorly at a lateral angel in order to accommodate tibial post 5150, as shown in FIG. 14, as compared to medial insert 5140B, as shown in FIG. 15, which is not configured to accommodate a tibial post. Additionally or alternatively, in some embodiments, the bearing surfaces of the one or more polyethylene inserts may be cross-linked, while the polyethylene comprising the post (modular or integral) may be non-cross-linked. Alternatively, in some embodiments, the polyethylene of the bearing surfaces and the inserts may be cross-linked.

In some embodiments, elements of an articular repair system may not be specifically designed with posterior stabilizing features for use in a PCL-sacrificing procedure but may be configured to accommodate the addition of posterior stabilizing features in the event that the PCL is sacrificed during the procedure. For example, the portion of the femoral component that will accommodate the box and/or cam can be standard, i.e., not-patient matched. In this manner, a stock of housings, receptacles or bars can be available in the operating room and added in case the surgeon sacrifices the PCL. In that case, the tibial insert can be exchanged for a tibial insert with a post mating with the box and/or cam for a posterior stabilized design.

In addition to the various posterior stabilizing features discussed above, and the features discussed in U.S. Patent Publication No. 2012-0209394, femoral and tibial implant component embodiments disclosed herein can include a number of other patient-adapted features and/or modifications. For example, in some embodiments, the femoral and/or tibial component can include one or more patient-adapted lugs. Such lugs can be configured, for example, to avoid interference with included posterior stabilizing features and/or to better accommodate forces relating to action on the posterior stabilizing features. Additionally or alternatively, a planned position, curvature, and/or slope of an articular surface of the femoral and/or tibial component may be adjusted to optimize one or more joint gap (e.g., flexion gap, extension gap) distances. For example, in some embodiments, an offset can be added to a posterior portion of one or more femoral condyles. The amount of such an offset may be based on patient-specific information, including, for example, a difference between subchondral bone and cartilage level at one or more locations and/or one or more tibial slopes. As another example, in some embodiments, the shape, dimensions, and/or curvature of one or more tibial and/or femoral articular surfaces may be adapted based on patient-specific information (e.g., the ligament information discussed above). In some such embodiment, the condylar surfaces may be adapted to guide and/or force a predetermined femoral rollback and/or rotation, optionally, with minimal or no influence of the post, box, and/or cams on the rollback and/or rotation.

### Collecting and modeling patient-specific data

As mentioned above, certain embodiments include implant components designed and made using patient-specific data that is collected preoperatively. The patient-specific data can include points, surfaces, and/or landmarks, collectively referred to herein as "reference points." In certain embodiments, the reference points can be selected and used to derive a varied or altered surface, such as, without limitation, an ideal surface or structure. For example, the reference points can be used to create a model of the patient's relevant biological feature(s) and/or one or more patient-adapted surgical steps, tools, and implant components. For example the reference points can be used to design a patient-adapted implant component having at least one patient-specific or patient-engineered feature, such as a surface, dimension, or other feature.

Reference points and/or data for obtaining measurements of a patient's joint, for example, relative-position measurements, length or distance measurements, curvature measurements, surface contour measurements, thickness measurements (in one location or across a surface), volume measurements (filled or empty volume), density measurements, and other measurements, can be obtained using any suitable technique. For example, one dimensional, two-dimensional, and/or three-dimensional measurements can be obtained using data collected from mechanical means, laser devices, electromagnetic or optical tracking systems, molds, materials applied to the articular surface that harden as a negative match of the surface contour, and/or one or more imaging techniques described above and/or known in the art. Data and measurements can be obtained non-invasively and/or preoperatively. Alternatively, measurements can be obtained intraoperatively, for example, using a probe or other surgical device during surgery.

In certain embodiments, imaging data collected from the patient, for example, imaging data from one or more of x-ray imaging, digital tomosynthesis, cone beam CT, non-spiral or spiral CT, non-isotropic or isotropic MRI, SPECT, PET, ultrasound, laser imaging, photo-acoustic imaging, is used to qualitatively and/or quantitatively measure one or more of a patient's biological features, one or more of normal cartilage, diseased cartilage, a cartilage defect, an area of denuded cartilage, subchondral bone, cortical bone, endosteal bone, bone marrow, a ligament, a ligament attachment or origin, menisci, labrum, a joint capsule, articular structures, and/or voids or spaces between or within any of these structures. The qualitatively and/or quantitatively measured biological features can include, but are not limited to, one or more of length, width, height, depth and/or thickness; curvature, for example, curvature in two dimensions (e.g., curvature in or projected onto a plane), curvature in three dimensions, and/or a radius or radii of curvature; shape, for example, two-dimensional shape or three-dimensional shape; area, for example, surface area and/or surface contour; perimeter shape; and/or volume of, for example, the patient's cartilage, bone (subchondral bone, cortical bone, endosteal bone, and/or other bone), ligament, and/or voids or spaces between them.

In certain embodiments, measurements of biological features can include any one or more of the illustrative measurements identified in Table 4.

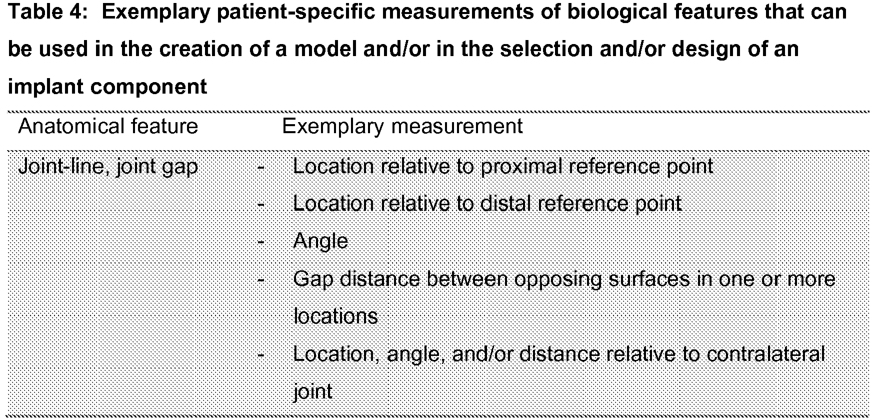

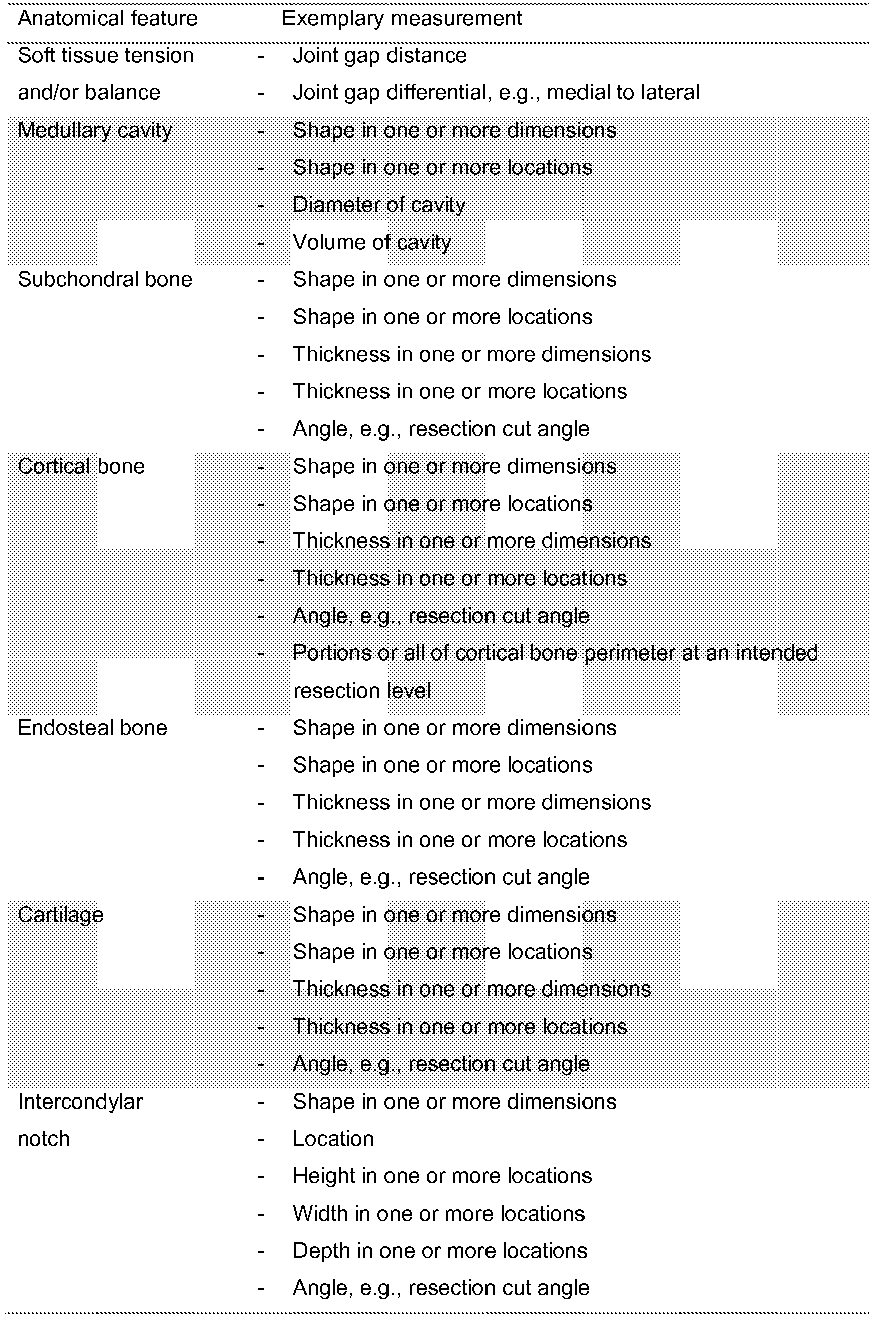

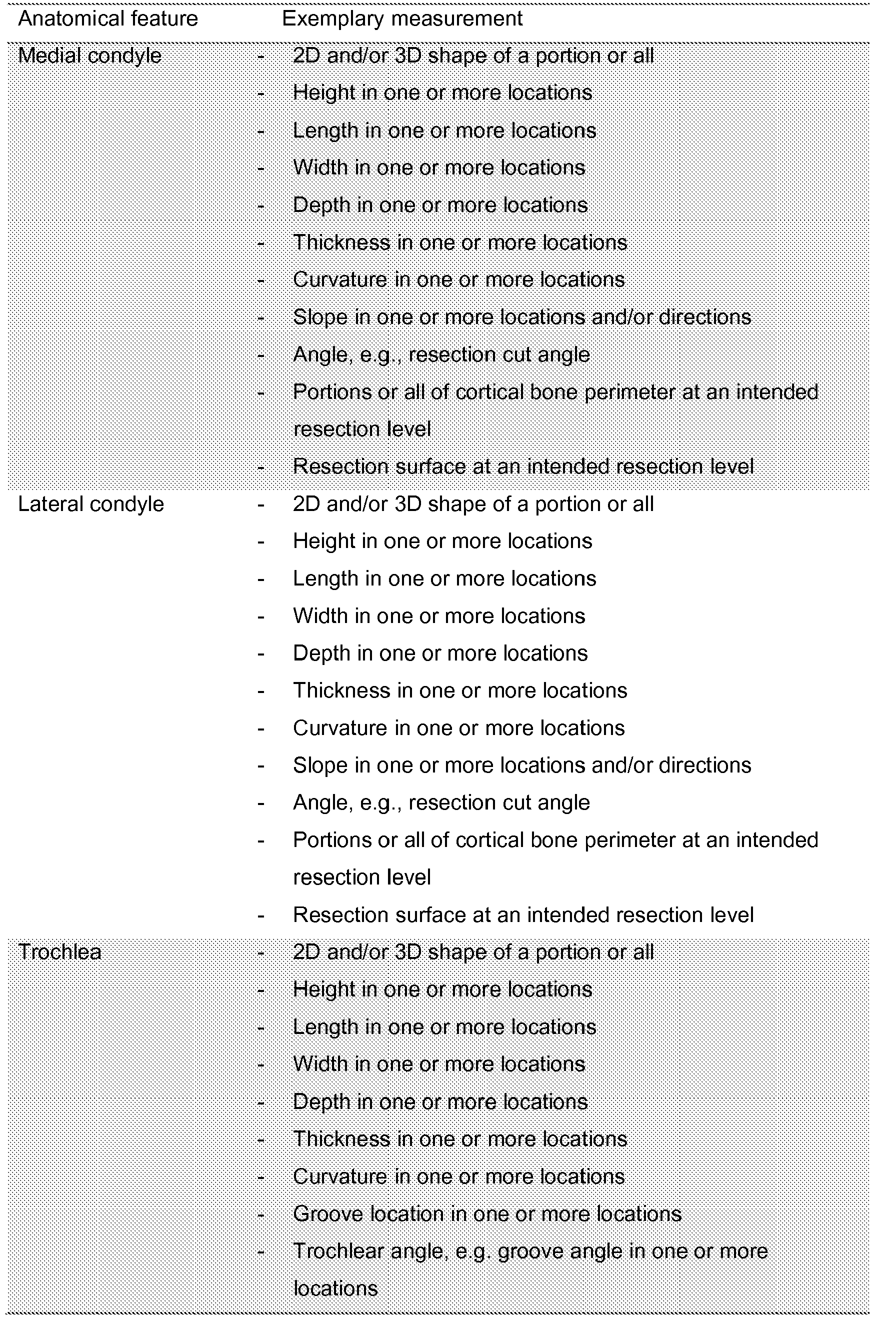

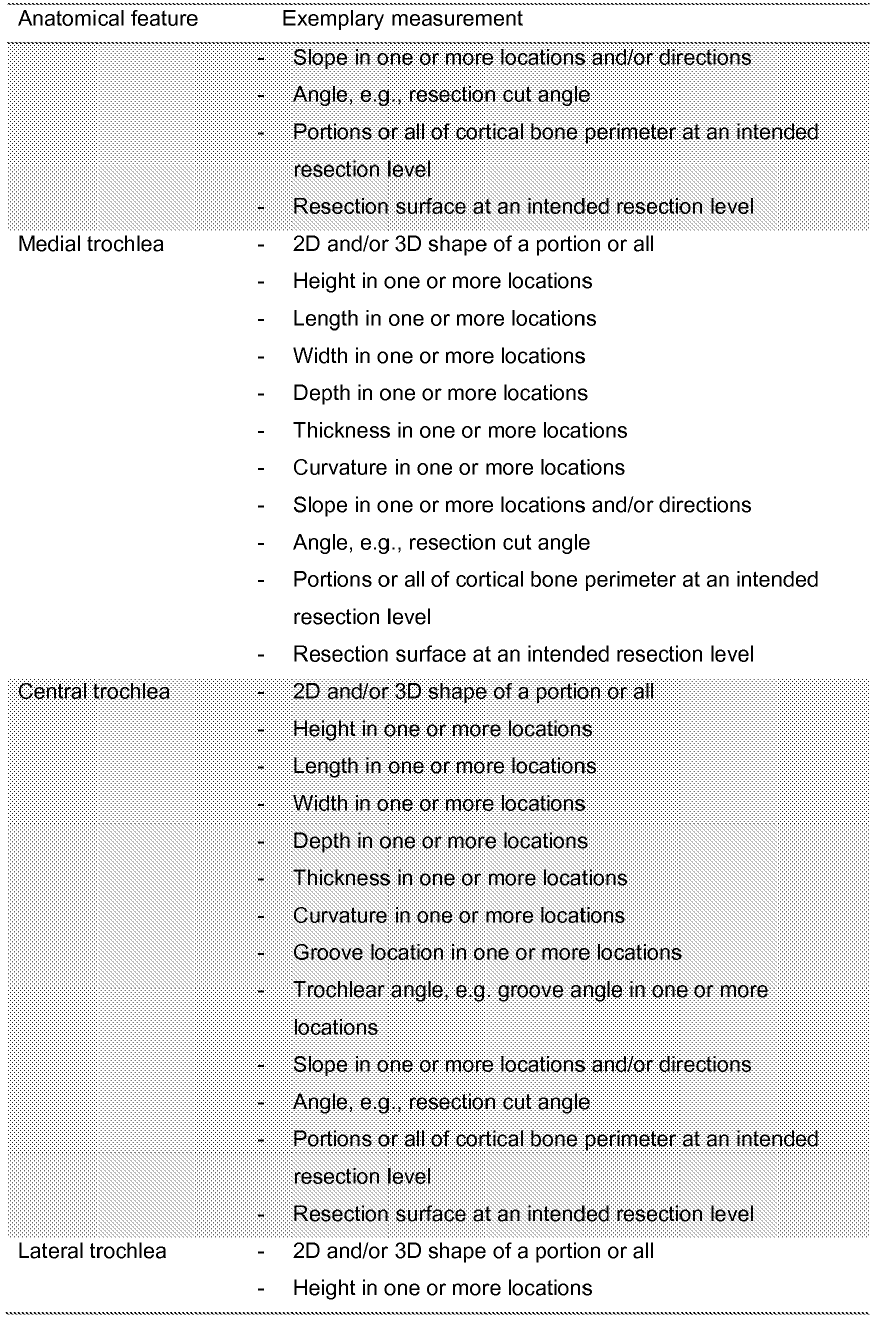

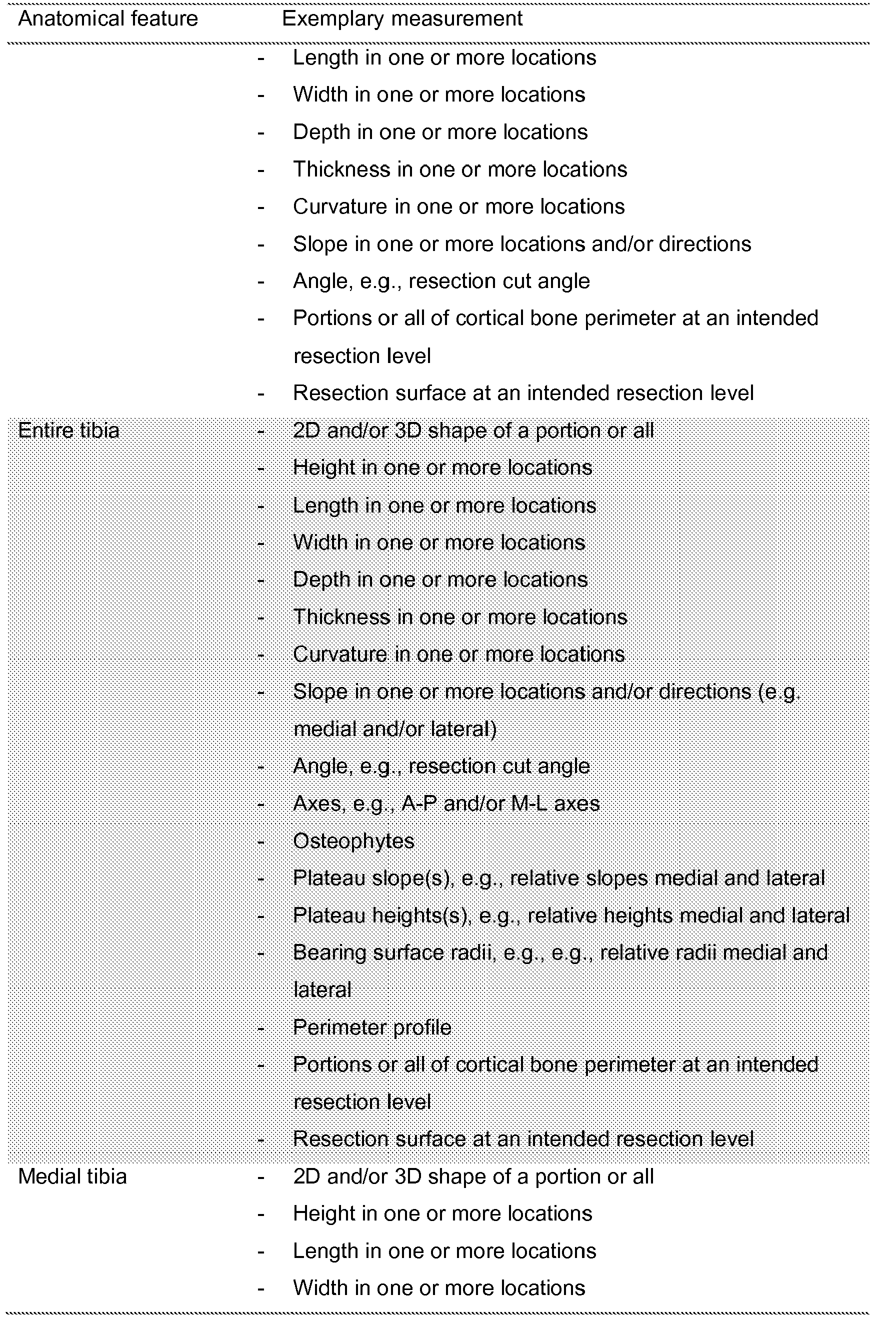

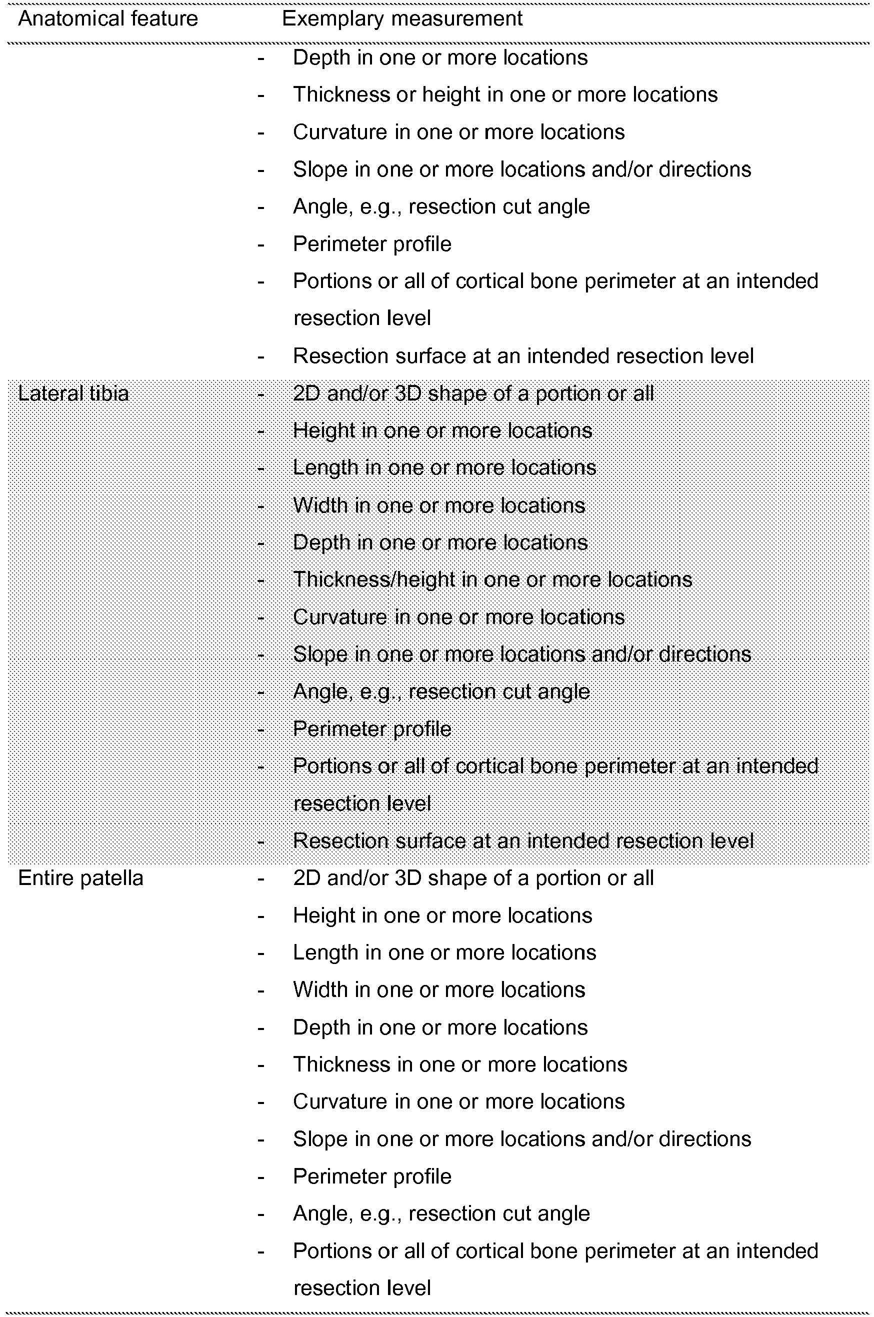

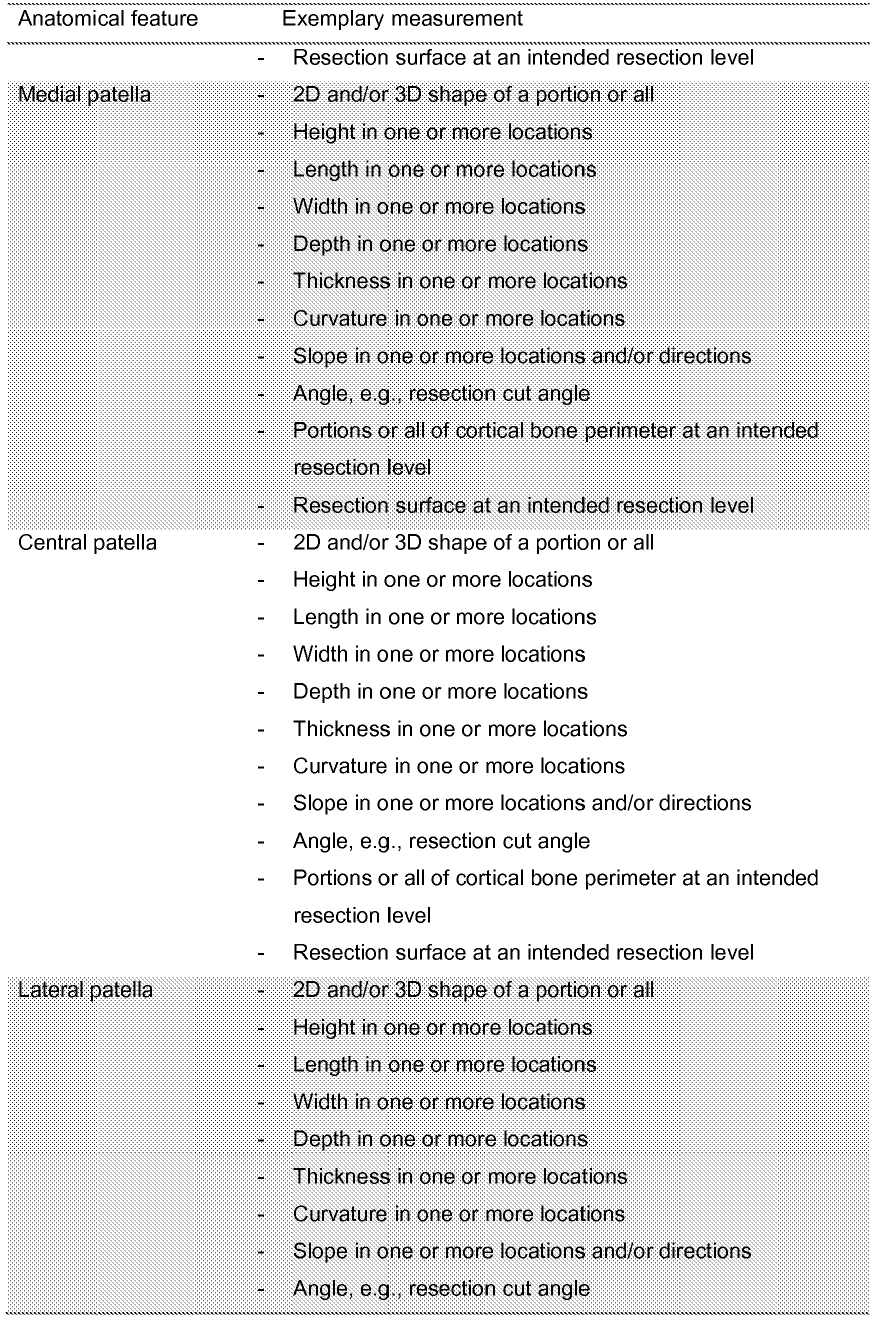

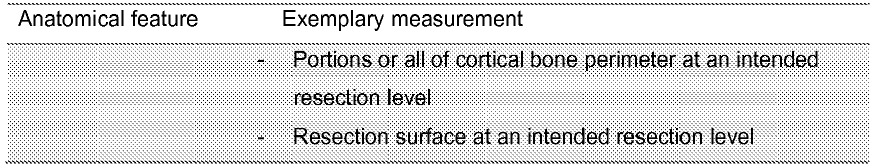

A single or any combination or all of the measurements described in Table 4 and/or known in the art can be used. Additional patient-specific measurements and information that can be used in the evaluation can include, for example, joint kinematic measurements, bone density measurements, bone porosity measurements, identification of damaged or deformed tissues or structures, and patient information, such as patient age, weight, gender, ethnicity, activity level, and overall health status. Moreover, the patient-specific measurements may be compared, analyzed or otherwise modified based on one or more "normalized" patient model or models, or by reference to a desired database of anatomical features of interest. For example, a series of patient-specific femoral measurements may be compiled and compared to one or more exemplary femoral or tibial measurements from a library or other database of "normal" femur measurements. Comparisons and analysis thereof may concern, but is not limited to one, more or any combination of the following dimensions: femoral shape, length, width, height, of one or both condyles, intercondylar shapes and dimensions, trochlea shape and dimensions, coronal curvature, sagittal curvature, cortical/cancellous bone volume and/or quality, etc., and a series of recommendations and/or modifications may be accomplished.

## Claims

1. A method of making a patient-adapted articular repair system for treatment of a knee joint of a patient, the knee joint including a femur and a tibia, the method comprising:
receiving patient-specific information;
deriving at least a portion of a shape of a joint-facing surface of a first condyle portion of a femoral implant component model from, at least in part, the patient-specific information;
deriving at least a portion of a shape of a joint-facing surface of a second condyle portion of the femoral implant component model from, at least in part, the patient-specific information;
deriving at least a portion of a shape of a first articular-bearing surface portion of a tibial implant component model from, at least in part, the patient-specific information;
deriving at least a portion of a shape of a second articular-bearing surface portion of the tibial implant component model from, at least in part, the patient-specific information;
aligning the femoral implant component model and the tibial implant component model disposed at a first flexion angle such that a bearing point of the joint-facing surface of the first condyle portion is aligned with a bearing point of the first articular-bearing surface;
determining a position of at least a first portion of a cam (5010, 5012) bearing surface relative to the first condyle portion and second condyle portion based, at least in part, on the position of the first condyle portion and/or the position of the second condyle portion relative to at least a portion of a bearing surface of a post portion (5150) of the tibial implant component, when the femoral implant component model and the tibial implant component model are disposed and aligned at the first flexion angle;
aligning the femoral implant component model and the tibial implant component model disposed at a second flexion angle such that a bearing point of the joint-facing surface of the first condyle portion is aligned with a bearing point of the first articular-bearing surface; and
determining a position of at least a second portion of the cam (5010, 5012) bearing surface relative to the first condyle portion and second condyle portion based, at least in part, on the position of the first condyle portion and/or the position of the second condyle portion relative to at least a portion of a bearing surface of the post portion (5150) of the tibial implant component, when the femoral implant component model and the tibial implant component model are disposed and aligned at the second flexion angle.

2. The method of claim 1, wherein the first condyle portion comprises a medial condyle portion of the femoral implant component model, the second condyle portion comprises a lateral condyle portion of the femoral implant component model, the first articular-bearing surface portion comprises a medial articular-bearing surface portion of the tibial implant component model, and the second articular-bearing surface portion comprises a lateral articular-bearing surface portion of the tibial implant component model.

3. The method of claim 1, wherein the first condyle portion comprises a lateral condyle portion of the femoral implant component model, the second condyle portion comprises a medial condyle portion of the femoral implant component model, the first articular-bearing surface portion comprises a lateral articular-bearing surface portion of the tibial implant component model, and the second articular-bearing surface portion comprises a medial articular-bearing surface portion of the tibial implant component model.

4. The method of claim 1, further comprising:
positioning the femoral implant component model and the tibial implant component model disposed at the second flexion angle such that a bearing point of the joint-facing surface of the first condyle portion is displaced posteriorly a first roll-back distance relative to a position of a bearing point of the first articular-bearing surface; and
determining a position of at least the second portion of the cam (5010, 5012) bearing surface relative to the first condyle portion and second condyle portion based, at least in part, on the position of the first condyle portion and/or the position of the second condyle portion relative to at least a portion of a bearing surface of the post portion (5150) of the tibial implant component, when the femoral implant component model and the tibial implant component model are disposed at the second flexion angle and the bearing point of the joint-facing surface is displaced posteriorly the first roll-back distance relative to the bearing point of the articular-bearing surface.

5. The method of claim 1, wherein the bearing point of the joint-facing surface of the first condyle portion comprises an inferior-most point of the joint-facing surface of the first condyle portion associated with a given flexion angle at which the femoral implant component model is disposed, and the bearing point of the first articular-bearing surface comprises an inferior-most point of the first articular-bearing surface.

6. The method of claim 1, further comprising:
deriving at least the portion of the shape of the first articular-bearing surface portion from, at least in part, the shape of at least a portion of the joint-facing surface of the first condyle portion; and
deriving at least the portion of the shape of the second articular-bearing surface portion from, at least in part, the shape of at least a portion of the joint-facing surface of the second condyle portion.

7. A patient-adapted articular repair system for treatment of a knee joint of a patient, the knee joint including a femur and a tibia, the system comprising:
a femoral implant component, the femoral implant component comprising:
a medial condyle portion, wherein at least a portion of a shape of a joint-facing surface of the medial condyle portion is derived, at least in part, from patient-specific information;
a lateral condyle portion, wherein at least a portion of a shape of a joint-facing surface of the lateral condyle portion is derived, at least in part, from patient-specific information; and
a cam portion (5010, 5012) substantially disposed between the medial condyle portion and the lateral condyle portion;
and
a tibial component, the tibial implant component comprising:
a medial articular-bearing surface portion, wherein at least a portion of a shape of the medial articular-bearing surface is derived, at least in part, from patient-specific information;
a lateral articular-bearing surface portion, wherein at least a portion of a shape of the lateral articular-bearing surface is derived, at least in part, from patient-specific information;
a post portion (5150), wherein the post portion includes at least one bearing surface and the post portion is substantially disposed between the medial articular bearing surface portion and the lateral articular bearing surface portion and extends substantially superiorly from the tibial implant component,
wherein the cam portion (5010, 5012) includes at least one bearing surface configured to engage at least a portion of the post bearing surface, when the femoral and tibial implant components are implanted on the femur and tibia, respectively, over at least a portion of a range of flexion of the knee joint,
wherein at least a portion of the bearing surface of the cam portion (5010, 5012) is positioned relative to the medial condyle portion and lateral condyle portion based, at least in part, on a patient-adapted position of the medial condyle portion and/or lateral condyle portion relative to at least a portion of the at least one bearing surface of the post portion, when at least a portion of the joint-facing surface of the medial condyle portion is engaged with at least a portion of the medial articular-bearing surface portion and/or at least a portion of the joint-facing surface of the lateral condyle portion is engaged with at least a portion of the lateral articular-bearing surface portion, at two or more flexion angles.

8. The method of claim 1 or the system of claim 7, wherein the joint-facing surface of the condyle portions each have a respective shape substantially in a sagittal plane that is derived from patient-specific information and a respective shape substantially in the coronal plane that is not patient-specific.

## Patentansprüche

1. Ein Verfahren zur Herstellung eines Pateinten-adaptierten Gelenk-Reparatursystems für die Behandlung eines Kniegelenks eines Patienten, wobei das Kniegelenk einen Femur und eine Tibia einschließt, das Verfahren umfassend:
das Erhalten Patienten-spezifischer Information;
das Ableiten mindestens eines Teils einer Form einer Gelenk-zugewandten Oberfläche eines ersten Kondylen-Teilstücks eines femoralen Implantat-Komponenten-Modells von, zumindest teilweise, der Patienten-spezifischen Information;
das Ableiten mindestens eines Teils einer Form einer Gelenk-zugewandten Oberfläche eines zweiten Kondylen-Teilstücks des femoralen Implantat-Komponenten-Modells von, zumindest teilweise, der Patienten-spezifischen Information;
das Ableiten mindestens eines Teils einer Form eines ersten Gelenk-tragenden Oberflächenteilstücks eines Tibia-Implantat-Komponenten-Modells von, zumindest teilweise, der Patienten-spezifischen Information;
das Ableiten mindestens eines Teils einer Form eines zweiten Gelenk-tragenden Oberflächenteilstücks des Tibia-Implantat-Komponenten-Modells von, zumindest teilweise, der Patienten-spezifischen Information;
Ausrichten des femoralen Implantat-Komponenten-Modells und des tibialen Implantat-Komponenten-Modells, die in einem ersten Beugungswinkel angeordnet sind, so dass der Lagerpunkt der Gelenk-zugewandten Oberfläche des ersten Kondylen-Teilstücks ausgerichtet ist an einem Lagerpunkt der ersten Gelenk-tragenden Oberfläche;
Bestimmung einer Position mindestens eines Teils einer Nockentragenden-Oberfläche (5010, 5012), relativ zum ersten Kondylen-Teilstück und zweiten Kondylen-Teilstück basierend auf, zumindest teilweise, der Position des ersten Kondylen-Teilstücks und/oder der Position des zweiten Kondylen-Teilstücks relativ zu mindestens einem Teil einer Trage-Oberfläche eines Pfosten-Teils (5150) der tibialen Implantat-Komponente, wenn das femorale Implantat-Komponenten-Modell und das tibiale Implantat-Komponenten-Modell angeordnet und ausgerichtet sind am ersten Beugungswinkel;
Ausrichten des femoralen Implantat-Komponenten-Modells und des tibialen Implantat-Komponenten-Modells, die in einem zweiten Beugungswinkel angeordnet sind, so dass ein Lagerpunkt einer Gelenk-zugewandten Oberfläche des ersten Kondylen-Teilstücks ausgerichtet ist an einem Lagerpunkt der ersten Gelenk-tragenden Oberfläche; und
Bestimmung einer Position mindestens eines zweiten Teils der Nockentragenden-Oberfläche relativ zum ersten Kondylen-Teilstück und zweiten Kondylen-Teilstück basierend auf, zumindest teilweise, der Position des ersten Kondylen-Teilstücks und/oder der Position des zweiten Kondylen-Teilstücks relativ zu mindestens einem Teil einer Trage-Oberfläche eines Pfosten-Teilstücks (5150) der tibialen Implantat-Komponente, wenn das femorale Implantat-Komponenten-Modell und das tibiale Implantat-Komponenten-Modell angeordnet und ausgerichtet sind am zweiten Beugungswinkel.

2. Das Verfahren nach Anspruch 1, worin das erste Kondylen-Teilstück ein mediales Kondylen-Teilstück des femoralen Implantat-Komponenten-Modells umfasst, das zweite Kondylen-Teilstück eines lateralen Kondylen-Teilstücks des femoralen Implantat-Komponenten-Modells umfasst, das erste Gelenk-tragende Oberflächen-Teilstück ein mediales Gelenk-tragendes Oberflächen-Teilstück des tibialen Implantat-Komponenten-Modells umfasst und das zweite Gelenk-tragende Oberflächen-Teilstück ein laterales Gelenk-tragendes Oberflächen-Teilstück des tibialen Implantat-Komponenten-Modells umfasst.

3. Das Verfahren nach Anspruch 1, worin das erste Kondylen-Teilstück ein laterales Kondylen-Teilstück des femoralen Implantat-Komponenten-Modells umfasst, das zweite Kondylen-Teilstück eines medialen Kondylen-Teilstücks des femoralen Implantat-Komponenten-Modells umfasst, das erste Gelenk-tragende Oberflächen-Teilstück ein laterales Gelenk-tragendes Oberflächen-Teilstücks des tibialen Implantat-Komponenten-Modells umfasst und das zweite Gelenk-tragende Oberflächen-Teilstück ein laterales Gelenk-tragendes Oberflächen-Teilstück des tibialen Implantat-Komponenten-Modells umfasst.

4. Das Verfahren nach Anspruch 1, ferner umfassend:
das Positionieren des femoralen Implantat-Komponenten-Modells und des tibialen Implantat-Komponenten-Modells, die in einem zweiten Beugungswinkel angeordnet sind, so dass ein Lagerpunkt einer Gelenk-zugewandten Oberfläche des ersten Kondylen-Teilstücks hinter einer ersten Zurückroll-Distanz verschoben ist relativ zu einer Position des Lagerpunktes der ersten Gelenk-tragenden Oberfläche; und
die Bestimmung einer Position mindestens eines zweiten Teils der Nockentragenden-Oberfläche (5010, 5012) relativ zum ersten Kondylen-Teilstück und zweiten Kondylen-Teilstück basierend auf, zumindest teilweise, der Position des ersten Kondylen-Teilstücks und/oder der Position des zweiten Kondylen-Teilstücks relativ zu mindestens einem Teil einer Trage-Oberfläche eines Pfosten-Teilstücks (5150) der tibialen Implantat-Komponente, wenn das femorale Implantat-Komponenten-Modell und das tibiale Implantat-Komponenten-Modell angeordnet sind im zweiten Beugungswinkel und der Lagerpunkt der Gelenk-zugewandten Oberfläche hinter eine erste Zurückroll-Distanz verschoben ist, relativ zum Lagerpunkt der Gelenk-tragenden Oberfläche.

5. Das Verfahren nach Anspruch 1, worin der Lagerpunkt der Gelenk-zugewandten Oberfläche auf dem ersten Kondylen-Teilstück einen untersten Punkt der Gelenk-zugewandten Oberfläche des ersten Kondylen-Teilstücks umfasst, assoziiert mit einem gegebenen Beugungswinkel, bei welchem das femorale Implantat-Komponenten-Modell angeordnet ist, und der Lagerpunkt der ersten Gelenk-tragenden Oberfläche einen untersten Punkt der ersten Gelenk-tragenden Oberfläche umfasst.

6. Das Verfahren nach Anspruch 1, ferner umfassend:
das Ableiten mindestens eines Teils einer Form des ersten Gelenkt-tragenden Oberflächen-Teilstücks von, zumindest teilweise, der Form mindestens eines Teils der Gelenk-zugewandten Oberfläche des ersten Kondylen-Teilstücks; und
das Ableiten mindestens eines Teils einer Form des zweiten Gelenkt-tragenden Oberflächen-Teilstücks von, zumindest teilweise, der Form mindestens eines Teils der Gelenk-zugewandten Oberfläche des zweiten Kondylen-Teilstücks.

7. Ein Patienten-adaptiertes Gelenkreparatursystem zur Behandlung des Kniegelenks eines Patienten, wobei das Kniegelenk einen Femur und eine Tibia einschließt, wobei das System umfasst:
eine femorale Implantat-Komponente, wobei die femorale Implantat-Komponente umfasst:
ein mediales Kondylen-Teilstück, worin mindestens ein Teil der Form der Gelenk-zugewandten Oberfläche des medialen Kondylen-Teilstücks abgeleitet ist, zumindest teilweise von Patienten-spezifischer Information;
ein laterales Kondylen-Teilstück, worin mindestens ein Teil der Form der Gelenk-zugewandten Oberfläche des lateralen Kondylen-Teilstücks abgeleitet ist, zumindest teilweise von Patienten-spezifischer Information; und
ein Nocken-Teilstück (5010, 5012), das hauptsächlich angeordnet ist zwischen dem medialen Kondylen-Teilstück und dem lateralen Kondylen-Teilstück;
und
eine tibiale Komponente, wobei die tibiale Komponente umfasst:
ein mediales Gelenk-tragendes Oberflächen-Teilstück, worin mindestens ein Teil einer Form der medialen Gelenk-tragenden Oberfläche abgeleitet ist, zumindest teilweise, von Patienten-spezifischer Information; und
ein laterales Gelenk-tragendes Oberflächen-Teilstück, worin mindestens ein Teil einer Form der lateralen Gelenk-tragenden Oberfläche abgeleitet ist, zumindest teilweise, von Patienten-spezifischer Information;
ein Pfosten-Teilstück (5150), worin das Pfosten-Teilstück mindestens eine Trage-Oberfläche einschließt und das Pfosten-Teilstück hauptsächlich angeordnet ist zwischen dem medialen Gelenk-tragenden Oberflächen-Teilstück und dem lateralen Gelenk-tragenden Oberflächen-Teilstück und sich hauptsächlich über der tibialen Implantat-Komponente erstreckt,
worin das Nocken-Teilstück (5010, 5012) mindestens eine Trage-Oberfläche einschließt, die so konfiguriert ist, dass sie mindestens einen Teil der Pfosten-Trage-Oberfläche einschließt, wenn die femoralen und tibialen Implantat-Komponenten über mindestens einen Teil des Beugungsbereichs des Kniegelenks auf Femur bzw. Tibia implantiert werden,
worin mindestens ein Teil der Trage-Oberfläche des Nocken-Teilstücks(5010, 5012) relativ zum medialen Kondylen-Teilstück und lateralen Kondylen-Teilstück positioniert ist, basierend auf, zumindest teilweise, einer Patienten-adaptierten Position des medialen Kondylen-Teilstücks und/oder lateralen Kondylen-Teilstücks relativ zu mindestens einem Teil der mindestens einen Trage-Oberfläche des Pfosten-Teilstücks, wenn mindestens ein Teil der Gelenk-zugewandten Oberfläche des medialen Kondylen-Teilstücks mit mindestens einem Teil des medialen Gelenk-tragenden Oberflächen-Teilstücks verbunden ist und/oder mindestens ein Teil der Gelenk-zugewandten Oberfläche des lateralen Kondylen-Teilstücks mit mindestens einem Teil des lateralen Gelenk-tragenden Oberflächen-Teilstücks verbunden ist, an zwei oder mehr Beugungswinkeln.

8. Das Verfahren nach Anspruch 1 oder das System von Anspruch 7, worin die Gelenkzugewandte Oberfläche der Kondylen-Teilstücke jeweils eine entsprechende Form hauptsächlich in einer sagittalen Ebene hat, die von Patienten-spezifischen Informationen abgeleitet ist und eine entsprechende Form, hauptsächlich in koronarer Ebene, die nicht Patienten-spezifisch ist.

## Revendications

1. Procédé de fabrication d'un système de réparation articulaire adapté au patient pour le traitement d'une articulation du genou d'un patient, l'articulation du genou comprenant un fémur et un tibia, le procédé comprenant :
la réception d'informations spécifiques du patient ;
l'obtention d'au moins une partie d'une forme d'une surface faisant face à l'articulation d'une première partie condyle d'un modèle de composant implant fémoral à partir, au moins en partie, d'informations spécifiques du patient ;
l'obtention d'au moins une partie d'une forme d'une surface faisant face à l'articulation d'une seconde partie condyle du modèle de composant implant fémoral à partir, au moins en partie, d'informations spécifiques du patient ;
l'obtention d'au moins une partie d'une forme d'une première partie surface d'appui articulaire d'un modèle de composant implant tibial à partir, au moins en partie, d'informations spécifiques du patient ;
l'obtention d'au moins une partie d'une forme d'une seconde partie surface d'appui articulaire du modèle de composant implant tibial à partir, au moins en partie, d'informations spécifiques du patient ;
l'alignement du modèle de composant implant fémoral et du modèle de composant implant tibial disposé à un premier angle de flexion de telle sorte qu'un point d'appui de la surface faisant face à l'articulation de la première partie condyle est aligné avec un point d'appui de la première surface d'appui articulaire ;
la détermination d'une position d'au moins une première partie d'une surface d'appui de came (5010, 5012) relativement à la première partie condyle et à la seconde partie condyle sur la base, au moins en partie, de la position de la première partie condyle et/ou de la position de la seconde partie condyle relativement à au moins une partie d'une surface d'appui d'une partie montant (5150) du composant implant tibial, lorsque le modèle de composant implant fémoral et le modèle de composant implant tibial sont disposés et alignés au premier angle de flexion ;
l'alignement du modèle de composant implant fémoral et du modèle de composant implant tibial disposés à un second angle de flexion de telle sorte qu'un point d'appui de la surface faisant face à l'articulation de la première partie condyle est aligné avec un point d'appui de la première surface d'appui articulaire ; et
la détermination d'une position d'au moins une seconde partie de la surface d'appui de came (5010, 5012) relativement à la première partie condyle et à la seconde partie condyle sur la base, au moins en partie, de la position de la première partie condyle et/ou de la position de la seconde partie condyle relativement à au moins une partie d'une surface d'appui de la partie montant (5150) du composant implant tibial, lorsque le modèle de composant implant fémoral et le modèle de composant implant tibial sont disposés et alignés au second angle de flexion.

2. Procédé selon la revendication 1, dans lequel la première partie condyle comprend une partie condyle médial du modèle de composant implant fémoral, la seconde partie condyle comprend une partie condyle latéral du modèle de composant implant fémoral, la première partie surface d'appui articulaire comprend une partie surface d'appui articulaire médiale du modèle de composant implant tibial, et la seconde partie surface d'appui articulaire comprend une partie surface d'appui articulaire latérale du modèle de composant implant tibial.

3. Procédé selon la revendication 1, dans lequel la première partie condyle comprend une partie condyle latéral du modèle de composant implant fémoral, la seconde partie condyle comprend une partie condyle médial du modèle de composant implant fémoral, la première partie surface d'appui articulaire comprend une partie surface d'appui articulaire latérale du modèle de composant implant tibial, et la seconde partie surface d'appui articulaire comprend une partie surface d'appui articulaire médiale du modèle de composant implant tibial.

4. Procédé selon la revendication 1, comprenant en outre :
le positionnement du modèle de composant implant fémoral et du modèle de composant implant tibial disposés à un second angle de flexion de telle sorte qu'un point d'appui de la surface faisant face à l'articulation de la première partie condyle est déplacé postérieurement sur une première distance de retour en arrière relativement à une position d'un point d'appui de la première surface d'appui articulaire ; et
la détermination d'une position d'au moins la seconde partie de la surface d'appui de came (5010, 5012) relativement à la première partie condyle et à la seconde partie condyle sur la base, au moins en partie, de la position de la première partie condyle et/ou de la position de la seconde partie condyle relativement à au moins une partie d'une surface d'appui de la partie montant (5150) du composant implant tibial, lorsque le modèle de composant implant fémoral et le modèle de composant implant tibial sont disposés au second angle de flexion et le point d'appui de la surface faisant face à l'articulation est déplacé postérieurement de la première distance de retour en arrière relativement au point d'appui de la surface d'appui articulaire.

5. Procédé selon la revendication 1, dans lequel le point d'appui de la surface faisant face à l'articulation de la première partie condyle comprend un point le plus inférieur de la surface faisant face à l'articulation de la première partie condyle associé à un angle de flexion donné auquel le modèle de composant implant fémoral est disposé, et le point d'appui de la première surface d'appui articulaire comprend un point le plus inférieur de la première surface d'appui articulaire.

6. Procédé selon la revendication 1, comprenant en outre :
l'obtention d'au moins la partie de la forme de la première partie surface d'appui articulaire à partir, au moins en partie, de la forme d'au moins une partie de la surface faisant face à l'articulation de la première partie condyle ; et
l'obtention d'au moins la partie de la forme de la seconde partie surface d'appui articulaire à partir, au moins en partie, de la forme d'au moins une partie de la surface faisant face à l'articulation de la seconde partie condyle.

7. Système de réparation articulaire adapté au patient pour le traitement d'une articulation du genou d'un patient, l'articulation du genou comprenant un fémur et un tibia, le système comprenant :
un composant implant fémoral, le composant implant fémoral comprenant :
une partie condyle médial, dans lequel au moins une partie d'une forme d'une surface faisant face à l'articulation de la partie condyle médial est obtenue, au moins en partie, d'informations spécifiques du patient ;
une partie condyle latéral, dans lequel au moins une partie d'une forme d'une surface faisant face à l'articulation de la partie condyle latéral est obtenue, au moins en partie, d'informations spécifiques du patient ; et
une partie came (5010, 5012) sensiblement disposée entre la partie condyle médial et la partie condyle latéral ; et
un composant tibial, le composant implant tibial comprenant :
une partie surface d'appui articulaire médiale, dans lequel au moins une partie d'une forme de la surface d'appui articulaire médiale est obtenue, au moins en partie, d'informations spécifiques du patient ;
une partie surface d'appui articulaire latérale, dans lequel au moins une partie d'une forme de la surface d'appui articulaire latérale est obtenue, au moins en partie, d'informations spécifiques du patient ;
une partie montant (5150), dans lequel la partie montant comprend au moins une surface d'appui et la partie montant est sensiblement disposée entre la partie surface d'appui articulaire médiale et la partie surface d'appui articulaire latérale et s'étend sensiblement vers le haut à partir du composant implant tibial,
dans lequel la partie came (5010, 5012) comprend au moins une surface d'appui configurée pour mettre en prise au moins une partie de la surface d'appui de montant, lorsque les composants implants fémoral et tibial sont implantés sur le fémur et le tibia, respectivement, sur au moins une partie d'une plage de flexion de l'articulation du genou,
dans lequel au moins une partie de la surface d'appui de la partie came (5010, 5012) est positionnée relativement à la partie condyle médial et à la partie condyle latéral sur la base, au moins en partie, d'une position adaptée au patient de la partie condyle médial et/ou condyle latéral relativement à au moins une partie de l'au moins une surface d'appui de la partie montant, lorsqu'au moins une partie de la surface faisant face à l'articulation de la partie condyle médial est en prise avec au moins une partie de la partie surface d'appui articulaire médiale et/ou au moins une partie de la surface faisant face à l'articulation de la partie condyle latéral est en prise avec au moins une partie de la partie surface d'appui articulaire latérale, à deux angles de flexion ou plus.

8. Procédé selon la revendication 1 ou système la revendication 7, dans lequel les surfaces faisant face à l'articulation des parties condyle ont chacune une forme respective sensiblement dans un plan sagittal qui est obtenue à partir d'informations spécifiques du patient et une forme respective sensiblement dans le plan coronal qui n'est pas spécifique du patient.
